(19) Europäisches Patentamt
European Patent Office
Office européen des brevets

(11) EP 0 529 444 B1

(12) EUROPÄISCHE PATENTSCHRIFT

(45) Veröffentlichungstag und Bekanntmachung des Hinweises auf die Patenterteilung: **16.04.1997 Patentblatt 1997/16**

(21) Anmeldenummer: **92113945.7**

(22) Anmeldetag: **17.08.1992**

(51) Int. Cl.6: **C07C 43/166**, C07C 43/174, C07C 43/178, C07C 57/38, C07C 59/48, C07C 59/52, C07C 59/64, C07C 59/84, C07C 69/618, C07C 69/734

(54) **2-Arylpropensäuren und ihre Verwendung bei der Herstellung von S-Ketoprofen**
2-Arylpropenic acids and their utilisation in the preparation of 5-ketoprofen
Acides 2-arylpropéniques et leur utilisation dans la préparation de 5-kétoprofen

(84) Benannte Vertragsstaaten:
**AT BE CH DE DK ES FR GB GR IT LI LU MC NL PT SE**

(30) Priorität: **30.08.1991 DE 4128787**

(43) Veröffentlichungstag der Anmeldung:
**03.03.1993 Patentblatt 1993/09**

(73) Patentinhaber: **BAYER AG**
**51368 Leverkusen (DE)**

(72) Erfinder:
- **Laue, Christian, Dr.**
  **D-4018 Langenfeld (DE)**
- **Schröder, Georg, Dr.**
  **D-5090 Leverkusen 1 (DE)**
- **Arlt, Dieter, Prof.Dr.**
  **D-5000 Köln 80 (DE)**

(56) Entgegenhaltungen:
**EP-A- 0 232 863** **DE-A- 2 613 817**

- **CHEMICAL ABSTRACTS, Bd. 92, Nr. 17, 28. April 1980, Columbus, Ohio, US; Zusammenfassung Nr. 146263j, Seite 542, Spalte 2; & JP-A-79 117 404**
- **CHEMICAL ABSTRACTS, Bd. 93, Nr. 1, 7. Juli 1980, Columbus, Ohio, US; Zusammenfassung Nr. 7865q, Seite 717, Spalte 2; & JP-A-79 148 761**
- **GAZZETTA CHEMICA ITALIANA, Bd. 110, Nr. 2-3, 1980, Seiten 123-127, V. SUJIC et al: "Enantioselective synthesis and absolute configuration of +)-alpha-(3-benzoylphenyl)pripionic acid"**
- **CHEMICAL ABSTRACTS, Bd. 93, Nr. 9, 1. September 1980, Columbus, Ohio, US; Zusammenfassung Nr. 94998b, Seite 617, Spalte 1; & JP-A-8 002 614**
- **BULLETIN OF THE CHEMICAL SOCIETY OF JAPAN, Bd. 63, 1990, Tokyo, JP, Seite 640-642, T. HIYAMA et al: "A facile, practical synthesis of 2-(6-methoxy-2-naphthyl)propenoic acid"**
- **CHEMISTRY LETTERS 1990, Tokyo, JP, Seite 523-526, T. KUSUMOTO et al: "Ferroelectric liquid crystalline compounds having a chiral center directly connected to the core aromatic ring"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 529 444 B1

## Beschreibung

Die vorliegende Erfindung betrifft neue 2-Arylpropensäuren, deren Derivate, Verfahren zu ihrer Herstellung sowie ihre Verwendung bei der Herstellung von S-Ketoprofen.

Racemisches Ketoprofen ist ein bekanntes Analgetikum und Antipyretikum. Wie bei anderen Arylpropionsäuren wie z.B. Naproxen und Ibuprofen ist bekannt, daß das S-Enantiomer gegenüber dem Racemat spezifische Vorteile wie Halbierung der Wirkdosis und Beschleunigung des Wirkungseintritts besitzt (vgl. 49 62 124).

Während für racemische Arylpropionsäuren und somit auch für racemisches Ketoprofen zahlreiche Synthesewege bekannt sind (vgl. z.B. J.P. Rieu et al, Tetrahedron, 42, 4095 (1986)), kann enantiomerenreines (S)-Ketoprofen nur sehr schwierig, in geringen Mengen und mit schlechten Ausbeuten hergestellt werden. Es wurde z.B. vorgeschlagen eine Racematspaltung von 2-(3-Benzylphenyl)-propionsäure mit anschließender Oxidation zu (S)-Ketoprofen durchzuführen (G. Comisso et al, Gazz. Chim. Italia 110, 123 (1980)). Die dort angegebene Ausbeute von 16 % ist bereits sehr niedrig. Bei einer Nacharbeitung dieses Verfahrens konnten nur noch schlechtere Ausbeuten erreicht werden.

In US-Pat. 3 641 127 wird eine Racematspaltung von 2-(2-Thiaxanthonyl)-propionsäuren mit nachfolgender mehrstufiger Überführung in (S)-Ketoprofen vorgeschlagen. Die hierbei erreichbaren Gesamtausbeuten sind noch wesentlich schlechter als bei dem obengenannten Verfahren von G. Comisso.

Durch eine chromatographische Diastereomerentrennung der Amide des Ketoprofens mit chiralen Aminen sind nach Verseifung der Amide nur mg-Mengen von (S)-Ketoprofen zugänglich (vgl. Abas et al., J. Pharmacol. Exp. Ther. 240 , 637 (1987)). Die in DE-A 3 824 353 beschriebene Racematspaltung mit Phenethylamin wurde ebenfalls versucht, führte aber nicht zum Erfolg.

Als weitere Möglichkeit wurde die asymmetrische Hydrierung von Dehydroketoprofen mit chiralen Rhodiumkatalysatoren vorgeschlagen. Diese Hydrierungen zeigen aber eine schlechte Enantioselektivität. In der obengenannten Publikation von G. Comisso wird z.B. eine Enantioselektivität von 60 % Enantiomerenüberschuß, nachfolgend e.e. genannt, angegeben. Selbst bei Durchführung einer Hydrierung in 2-Phasen-Systemen wie in EPA 419 312 beschrieben, ergeben sich nur Enantioselektivitäten von 71 % e.e.. Ein wesentlicher Nachteil der dort genannten asymmetrischen Hydrierungen ist ihre schwierige großtechnische Durchführung, die nur mit großem Aufwand erfolgen kann. Weiterhin sind die einzusetzenden chiralen Katalysatoren äußerst schwer herstellbar.

Zusammenfassend ist festzustellen, daß bisher kein technisches Verfahren bekannt ist, das die Produktion größerer Mengen von (S)-Ketoprofen ermöglicht.

Die Erfindung betrifft neue (2)-Arylpropensäuren der allgemeinen Formel (II)

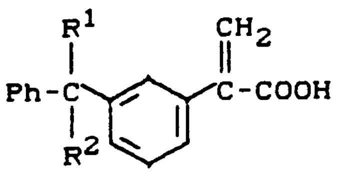

(II)

in welcher

$R^1$ und $R^2$ gleich oder verschieden sind und jeweils für Wasserstoff, Hydroxy, Alkoxy mit bis zu 10 C-Atomen, Cycloalkoxy mit 5 bis 10 C-Atomen, Aralkoxy mit 7 bis 12 C-Atomen oder Aryloxy mit 6 bis 10 C-Atomen, der gegebenenfalls substituiert ist durch Alkyl oder Alkoxy mit jeweils 1 bis 5 C-Atomen, stehen

oder

für die Gruppe $O_2CR^3$ stehen, wobei $R^3$ für Wasserstoff, Alkyl mit bis zu 10 C-Atomen, Cycloalkyl mit 5 bis 10 C-Atomen, Aralkyl mit 7 bis 12 C-Atomen oder Aryl mit 6 bis 10 C-Atomen, gegebenenfalls substituiert mit Alkyl oder Alkoxy mit jeweils 1 bis 5 C-Atomen steht,

oder

für die Gruppe $OSiR^4R^5R^6$ stehen, wobei $R^4$, $R^5$ und $R^6$ unabhängig voneinander jeweils die für $R^3$ angegebene Bedeutung haben

oder

für die Gruppe $NR^7R^8$ stehen, wobei $R^7$ und $R^8$ unabhängig voneinander die für $R^3$ angegebene Bedeutung haben

oder

für eine 2-Tetrahydropyrylgruppe stehen

oder

$R^1$ und $R^2$ gemeinsam für eine Gruppierung der Struktur

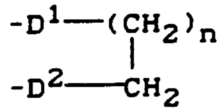

stehen, worin

- $D^1$ und $D^2$ unabhängig voneinander jeweils Sauerstoff, Schwefel oder die Gruppe $NR^3$ bedeuten, wobei $R^3$ die oben angegebene Bedeutung hat und
- n für 1, 2 oder 3 steht.

Von besonderem Interesse sind Verbindungen der allgemeinen Formel (II), in welcher

- R und $R^2$ jeweils für Wasserstoff, Hydroxyl, Alkoxy mit 1 bis 4 C-Atomen, Benzyloxy, Acetyloxy, Benzoyloxy, Tri(alkyl)silyl mit jeweils 1 bis 4 C-Atomen in den Alkylresten, Dialkylamino mit jeweils 1 bis 4 C-Atomen in den Alkylresten oder für 2-Tetrahydropyryl stehen

oder

- $R^1$ und $R^2$ zusammen für die Gruppe $-O(CH_2)_n-CH_2-D^2$-stehen, wobei n für 1, 2 oder 3 steht und $D^2$ für Sauerstoff oder Alkylamin mit 1 bis 4 C-Atomen steht.

Besonders bevorzugt sind die folgenden Vertreter der Verbindungen der allgemeinen Formel (II):

der Alkohol ($R^1$ = OH, $R^2$ = H),
die Alkylether ($R^1$ = $OCH_3$, $OC_2H_5$, $R^2$ = H),
Dioxoverbindung ($R^1$ und $R^2$ = $-O(CH_2)_2O-$ oder $-O(CH_2)_3O-$),
das Ketal ($R^1$ und $R^2$ = $(-OC_2H_5)_2$),
Ester ($R^1$ = $OOCCH_3$, $R^2$ = H),
Trialkylsiloxyl-Ether ($R^1$ = $(CH_3)_3SiO$, $R^2$ = H).

Die Erfindung betrifft weiterhin die Verwendung der neuen Verbindungen der allgemeinen Formel (II) zur Herstellung von (S)-Ketoprofen, dadurch gekennzeichnet, daß man die Verbindungen der allgemeinen Formel (II) in Gegenwart eines Katalysators der aus einem chiralen Übergangs-Metallkomplex besteht und gegebenenfalls in Gegenwart von inerten organischen Lösungsmitteln zu Verbindungen der allgemeinen Formel (I)

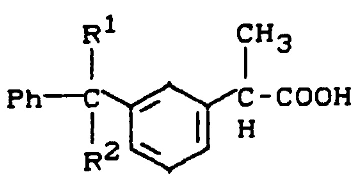

(I)

in welcher

- $R^1$ und $R^2$ die oben angegebene Bedeutung haben, aber nicht gleichzeitig für Wasserstoff stehen,

hydriert und anschließend die Gruppe $CR^1R^2$ nach üblichen Methoden in eine Ketogruppe umwandelt.

Als katalytisch einsetzbare chirale Übergangs-Metallkomplexe seien bevorzugt Rhodium-, Ruthenium- und Iridium-Komplexe mit chiralen Bisphosphinen genannt.

Die hierfür verwendbaren Bisphosphine sind bekannt oder können nach bekannten Methoden hergestellt werden (vgl. EPA 403 188).

Die Ruthenium-, Iridium- und Rhodium-Komplexe sind ebenfalls bekannt oder können nach bekannten üblichen Methoden hergestellt werden (vgl. EPA 369 691, 366 390, 245 959, 398 132 oder Tetrahedron Letters 32, 3101 (1991) oder Tetrahedron Asymmetry 1, 859 (1990)).

Als Lösungsmittel für die Hydrierungen finden Verwendung niedere Alkohole wie Methanol, Ethanol oder Toluol, Benzol, Tetrahydrofuran oder ihre Mischungen, vorzugsweise Methanol, die Konzentrationen betragen 0,5 bis 20 l per mol Edukt.

Der Katalysator wird benutzt in einer Konzentration von 0,05 bis 0,0001 mol, bevorzugt in einer Konzentration von 0,01 bis 0,001 mol, per mol Arylpropensäure.

Im Falle der Rhodium-Katalysatoren werden besonders bevorzugt 0,1 bis 1,1 eq tert. Amine zugegeben. Der Wasserstoffdruck der Reaktion beträgt 1 bis 200 atm, vorzugsweise 10 bis 100 atm, die Temperatur 5 bis 100°C, vorzugsweise 10 bis 70°C und die Reaktionsdauer 10 min bis 52 h.

Die Hydrierung wird bevorzugt in einem entgasten inerten Lösungsmittel unter Sauerstoffausschluß unter Rühren oder Schütteln vorgenommen.

Die Erfindung betrifft ebenfalls mehrere Verfahren zur Herstellung der Verbindungen der allgemeinen Formel (II)

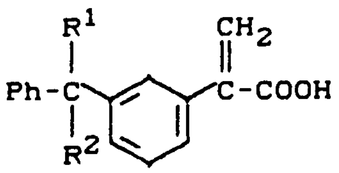

(II)

in welcher

$R^1$ und $R^2$ die oben angegebene Bedeutung haben,

die dadurch gekennzeichnet sind, daß man

A) Verbindungen der allgemeinen Formel (III)

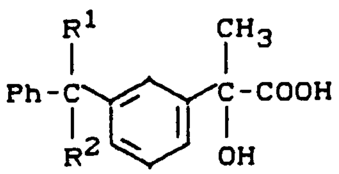

(III)

in welcher

$R^1$ und $R^2$ die oben angegebene Bedeutung haben,

nach üblichen Methoden dehydratisiert
oder
B) Verbindungen der Formel (IV)

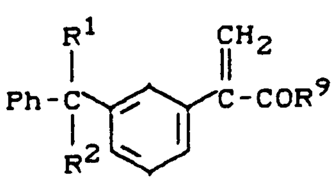

(IV)

in welcher

$R^1$ und $R^2$ die oben angegebene Bedeutung haben und
$R^9$ für Alkoxy mit 1 bis 10 C-Atomen, Cycloalkoxy mit 5 bis 10 C-Atomen, Aralkoxy mit 7 bis 12 C-Atomen oder Aryloxy mit 6 bis 10 C-Atomen, das gegebenenfalls durch Alkyl oder Alkoxy mit jeweils 1 bis 5 C-Atomen substituiert ist, steht,

nach üblichen Methoden hydrolysiert.

Variante A

Die Abspaltung des Wassers gemäß Verfahrensvariante A) erfolgt vorzugsweise durch Einwirkung von Hitze und/oder Säure gegebenenfalls in Gegenwart eines Lösungsmittels bei Temperaturen zwischen 50 und 250°C, vorzugsweise zwischen 70 und 150°C, insbesondere beim Siedepunkt des verwendeten Lösungsmittels.

Als Lösungsmittel finden vorzugsweise Ether, Kohlenwasserstoffe, chlorierte Kohlenwasserstoffe, Dimethylformamid, Dimethylsulfoxid oder ihre Gemische Verwendung. Als Säuren werden vorzugsweise Mineralsäuren, Sulfonsäuren oder Carbonsäuren verwandt. Besonders bevorzugt ist 5 bis 30 %ige Salzsäure, Polyphosphorsäure, 2 bis 15 % Schwefelsäure in Dioxan oder Lösungen von 0,1 bis 5 % p-Toluolsulfonsäure in Toluol oder Benzol.

Variante B

Die Verseifung der Verbindungen der allgemeinen Formel (IV) gemäß Verfahrensvariante B) erfolgt nach üblichen Methoden gegebenenfalls in Gegenwart von inerten organischen Lösungsmitteln bei Temperaturen zwischen 20 und 150°C, insbesondere zwischen 40 und 120°C.

Ein weiterer Gegenstand der Erfindung sind Verbindungen der allgemeinen Formel (IV), worin $R^1$, $R^2$ und $R^9$ die zuvor beschriebenen Bedeutungen besitzen, sowie ein Verfahren zu ihrer Herstellung, das dadurch gekennzeichnet ist, daß Verbindungen der allgemeinen Formel (V)

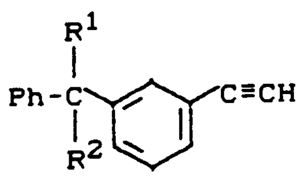

(V)

worin

$R^1$ und $R^2$ die obengenannte Bedeutung haben,

in Gegenwart von Palladium auf Aktivkohle als Katalysator und Kohlenmonoxid bei Drucken zwischen 1 und 200 bar mit einem Alkohol gemäß der Definition $HR^9$, wobei $R^9$ die oben angegebene Bedeutung hat, unter Zugabe von Mineralsäure, gegebenenfalls in Anwesenheit eines inerten aprotischen Lösungsmittels bei Temperaturen zwischen 25 und 160°C umsetzt.

Aus der Literatur sind bereits Methoden zur Herstellung von 2-Propensäureestern und 2-Arylpropensäuren durch Reaktion von Alkinen mit Kohlenmonoxid in Gegenwart von Katalysatoren bekannt (siehe T. Kusumoto, et al., Chem. Lett., 1990, 523; T. Hiyama, et al., Bull. Chem. Soc. Jpn., 63, 640 (1990)). Als Katalysatoren werden Palladium-Schwarz, Metallsalze oder Metallsalz-Komplexe, die z.B. Triarylphosphine enthalten, eingesetzt, die hergestellt werden müssen oder durch gelösten Wasserstoff zur teilweisen Reduktion der eingesetzten Alkine zu Alkenen oder durch Abtrennung der Phosphine vom Reaktionsgemisch zu Schwierigkeiten führen.

Weiterhin sind aus der Literatur Reaktionen von Alkinen mit stöchiometrischen Mengen von Metallcarbonylen (siehe E.R.H. Jones, et al., J. Chem. Soc., 230 (1958); C.W. Bird, Chem. Rev., 283, (1962)) bekannt, die jedoch wegen der Giftigkeit der Metallcarbonyle keine echte Alternative zu den obigen Methoden darstellen.

Für die Umsetzung von Alkinen zu den 2-Arylpropensäuren bzw. deren Estern werden für eine vereinfachte Anwendung heterogene Katalysatoren gewünscht, die eine sehr einfache Abtrennung vom Reaktionsgemisch unter Vermeidung aller zuvor genannter Probleme gestatten. Aus der Literatur ist bekannt, daß Palladium auf Aktivkohle ein für diese Reaktion ungeeigneter Katalysator ist (G.P. Giusoli, et al., Gazz. Chim. Ital., 115, 691 (1985)). Es war daher um so überraschender, daß sich Arylpropensäuren mit Palladium auf Aktivkohle in guten Ausbeuten herstellen ließen.

Die Menge des Palladium-Aktivkohle-Katalysators beträgt vorzugsweise 0,1 bis 25 Gew.-%, vorzugsweise 1 bis 20 Gew.-% und insbesondere 5 bis 15 Gew.-%, bezogen auf die eingesetzte Verbindung der allgemeinen Formel (V).

Die Umsetzung erfolgt vorzugsweise bei Drucken zwischen 5 und 150 bar, insbesondere zwischen 10 und 100 bar.

Als besonders geeignete Alkohole seien niedere Alkohole mit bis zu 6, insbesondere mit bis zu 4 C-Atomen, ganz besondere Methanol und Ethanol genannt. Die Mineralsäure wird vorzugsweise in Mengen von 0,1 bis 10 mol, insbesondere 0,2 bis 5 mol, besonders 0,5 bis 2 mol, jeweils bezogen auf das eingesetzte Alkin der allgemeinen Formel (V), zugegeben. Besonders bevorzugte Mineralsäuren sind Halogen-Wasserstoffsäuren wie Brom-, Chlor- und Iodwasserstoffsäure.

Es hat sich als vorteilhaft erwiesen 0,1 bis 10 mol, vorzugsweise 0,2 bis 5 mol, besonders 0,5 bis 2 mol Alkalimetallhalogenide (bezogen auf eingesetztes Alkin) einzusetzen. Neben Chloriden und Bromiden seien Iodide bevorzugt genannt.

Die Reaktionstemperatur beträgt vorzugsweise 40 bis 140°C, insbesondere 50 bis 120°C bei Reaktionszeiten zwischen 1 und 48 Stunden, insbesondere zwischen 4 und 24 Stunden.

Die erhaltenen Produkte der allgemeinen Formel (IV) werden nach üblichen Methoden wie z.B. Chromatographie, Destillation und Kristallisation gereinigt.

Ein weiterer Gegenstand der Erfindung sind Verbindungen der allgemeinen Formel (III)

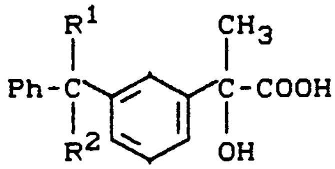

(III)

in welcher

$R^1$ und $R^2$ die oben angegebene Bedeutung haben.

Die Herstellung der Verbindung der allgemeinen Formel (III) erfolgt, indem man Grignardverbindungen der allgemeinen Formel (VI)

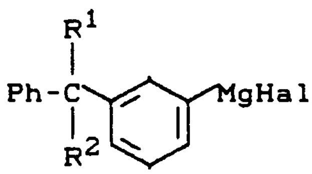

(VI)

in welcher

$R^1$ und $R^2$ die oben angegebene Bedeutung haben und
Hal für Fluor, Chlor, Jod oder Brom, insbesondere für Brom und Chlor steht,

mit einem Metall-pyruvat der allgemeinen Formel (VII)

$$Me^{\oplus}\ {}^{\ominus}OCC\text{-}CO\text{-}CH_3 \qquad \text{(VII)}$$

in welcher

Me für Alkali, Erdalkali (ein halbes Äquivalent) oder MgBr steht,

in Gegenwart von organischen Lösungsmitteln bei Temperaturen zwischen -10°C und +120°C umsetzt.

Die als Ausgangsverbindungen einzusetzenden Grignardverbindungen der allgemeinen Formel (VI) sind bekannt oder können nach bekannten Methoden hergestellt werden (vgl. DE-A-26 13 817).

Die Metallsalze der Brenztraubensäure der allgemeinen Formel (VII) sind ebenfalls bekannt und kommerziell erhältlich. Von besonderer Bedeutung ist die Verwendung des Natriumsalzes der Brenztraubensäure. Neben den Metallsalzen können auch die entsprechenden Ammoniumsalze eingesetzt werden.

Die Umsetzung erfolgt vorzugsweise durch Mischen der Grignardverbindung der allgemeinen Formel (VI) mit Verbindungen der allgemeinen Formel (VII), die gegebenenfalls in einem Ether oder einem inerten Kohlenwasserstoff oder einem Gemisch von beiden gelöst oder suspendiert sind. Besonders geeignet sind Diethylether, Tetrahydrofuran und Dimethoxyethan. Die Reaktion erfolgt bevorzugt bei Temperaturen zwischen 0 und 100°C.

Die erfindungsgemäße Umwandlung von Verbindungen der allgemeinen Formel (I) in (S)-Ketoprofen erfolgt nach üblichen Methoden je nach Art der Substituenten $R^1$ und $R^2$ die variiert werden können. Folgende Oxidationsmethoden seien beispielhaft genannt:

Für die direkte Oxidation der Ether von sekundären Benzylalkoholen zu Ketonen seien beispielhaft genannt: Nitro-

niumtetrafluoroborat, Natriumbromat/$(NH_4)_2Ce(IV)(NO_3)_6$, $IF_5$, $UF_6$, Trityltetrafluoroborat, Chromylchlorid.

Die Alkylether ($R^1 = OR^3$, $R^2 = H$) werden bevorzugt erfindungsgemäß racemisierungsfrei durch Erhitzen in einem inerten Lösungsmittel wie z.B. Tetrachlorkohlenstoff mit 1-2 eq, bevorzugt mit 1,05 bis 1,2 eq N-Brom-Succinimid oder N-Chlor-Succinimid, bevorzugt mit N-Brom-Succinimid unter Zugabe der üblichen Radikalkettenstarter wie Benzoylperoxid oder Azo-bis-isobutyronitril oder unter Bestrahlung mit Licht, bevorzugt mit Benzoylperoxid oder unter Bestrahlung mit Licht, oxidiert.

Die Oxidation mit Natriumbromat/$(NH_4)_2Ce(IV)(NO_3)_6$ ist ebenfalls geeignet.

Alternativ kann zunächst in einer literaturbekannten Etherspaltung der Alkohol ($R^1CR^2$ = HCOH) hergestellt werden, der nach unten beschriebenen Verfahren dann weiter zu Ketoprofen oxidiert wird.

Alternativ kann in einem literaturüblichen Verfahren hydrogenolytisch (z.B. Pd/C; $H_2$) zur Benzylgruppe ($R^1CR^2$ = HCH) reduziert werden, die dann nach unten beschriebenen Verfahren zu Ketoprofen oxidiert wird.

Die Methylenverbindung ($R^1$, $R^2$ = H) kann mit $KMnO_4$ und einem Katalysator, z.B. Co-Naphthenat oder anderen Übergangsmetall-Salzen oder mit $O_2$/Co-Naphthenat zu Ketoprofen (I) oxidiert werden.

Ebenfalls geeignet ist eine Oxidation mit $CrO_3$ in Eisessig bei 10 bis 50°C.

Für die racemisierungsfreie Oxidation des Alkohols ($R^1$ = OH, $R^2$ = H) zu (S)-Ketoprofen (I) verwendet man bekannte Oxidationsmittel wie Cr(VI)-Salze, Selendioxid, Brom, Natriumbromat/$(NH_4)_2Ce(IV)(NO_3)_6$, bevorzugt jedoch Brom.

Die Oxidation wird vorzugsweise bei Raumtemperatur in einem chlorierten Kohlenwasserstoff oder einem Alkohol, bevorzugt in Methanol mit 1-10 eq Brom, bevorzugt mit 1,5-3 eq durchgeführt.

Ketale der allgemeinen Formel II ($R^1$, $R^2$ = $-O-CH_2-(CH_2)_nO-$) n = 1, 2 und können nach üblichen Verfahren mit Sulfonsäuren oder wäßriger Mineralsäure racemisierungsfrei in Ketoprofen (I) überführt werden.

Ester ($R^1$ = H, $R^2$ = $OOCR^3$) können nach üblichen Verfahren in die Alkohole umgewandelt werden.

Tetrahydropyryl-ether und Silylether ($R^2$ = $Me_3SiO$, $R^2$ = H) können nach üblichen Verfahren in die Alkohole umgewandelt werden.

Alternativ zu den oben angegebenen Methoden können Benzylether ($R^1$ = $O_2CR^3$, $R^2$ =H) oder Ether ($R^1$ = $OR^9$, $R^2$ =H) hydrogenolytisch (Houben-Weyl, Band 4/1c S. 400-401) in die Benzylverbindung ($R^1$ = H, $R^2$ = H) umgewandelt werden, die nach oben beschriebenen Methoden in Ketoprofen überführt werden können.

Bei der einfachen Übertragung der asymmetrischen Hydrierung von Naproxen, die in DEA 0 272 787 beschrieben wird, auf die Reaktion von Dehydroketoprofen zu Ketoprofen wurde überraschenderweise nur ein sehr schlechter Enantiomerenüberschuß von 47 % e.e. gefunden. Es ist daher ausgesprochen überraschend, daß sich die Verbindungen der Formel (II) mit so guten Enantioselektivitäten hydrieren lassen.

Das erfindungsgemäße Verfahren ermöglicht in technischem Maßstab größere Mengen der nach den bisher bekannten Verfahren nur schwer zugänglichen Verbindung (S)-Ketoprofen herzustellen.

Das nach dem erfindungsgemäßen Verfahren hergestellte (S)-Ketoprofen wird als Analgetikum und Antipyretikum (US 49 62 124) verwendet. Dabei hat das reine (S)-Enantiomer verbesserte Eigenschaften gegenüber dem Racemat.

Die Herstellung von (S)-Ketoprofen wird durch die Beispiele 1, 2 und 3 gestützt.

Die Herstellung der Verbindungen der allgemeinen Formel (I) wird durch die Beispiele 4, 5 und 6 gestützt.

Die Herstellung von Verbindungen der allgemeinen Formel (II) wird durch die Beispiele 7, 8, 9 und 10 gestützt.

Die Herstellung von Verbindungen der Formel (III) wird durch die Beispiele 11, 12 und 13 gestützt.

Die Herstellung von Verbindungen der Formel (IV) wird durch die Beispiele 14, 15 und 16 gestützt.

Falls nicht ausdrücklich anders angegeben steht die Abkürzung BINAP für die Formel

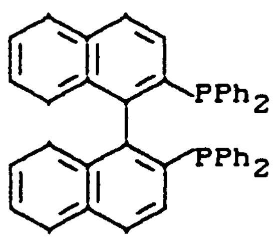

Der Begriff Enantiomerenüberschuß ist wie folgt definiert; die Angaben erfolgen in %:

$$\text{e.e.} = \frac{\text{Masse des Enantiomeren A - Masse des Enantiomeren B}}{\text{Masse des Enantiomeren A + Masse des Enantiomeren B}} \times 100$$

Die Bestimmung des Enantiomerenüberschusses erfolgte nach literaturbekannten Methoden (EP-A-O- 379917) durch HPLC an chiralen Phasen.

Vergleichsbeispiel

Asymmetrische Hydrierung von 2-((3-Benzoyl)-phenyl)-propensäure (Dehydroketoprofen)

In einen 100 ml Autoklaven wird eine Lösung von 0,3 g (1,2 mmol) 2-((3-Benzoyl)-phenyl)-propensäure (Gazz. Chim. Ital. 110, 123 (1980)) in 5 ml sauerstoff-freiem Methanol und 10 mg (0,012 mmol) Ru(S)-BINAP-$(O_2CCH_3)_2$ (EPA 0 245 959) unter Schutzgas eingefüllt. Nach 48 h Reaktion unter 100 atm Wasserstoff wird das Lösungsmittel entfernt, der Rückstand mit Essigester aufgenommen und zur Entfernung des Katalysators über Kieselgel filtriert.

Ausbeute: 0,287 g (95 %).

Enantiomerenüberschuß: 47 % e.e.

$^1$H-NMR (250 MHz, $CDCl_3$) $\delta$ = (ppm) 1,58 (d, J=7,5 Hz; 3H, $CH_3$), 3,85 (q, J=7 Hz; 1H, $CHCH_3$), 7,4-7,95 (m; 9H, $H_{aromat.}$).

Beispiel 1

Oxidation von 2-(3-Benzyl-phenyl)-propansäure

1 g (4,16 mmol) des Produktes aus Beispiel 4 und 2,5 g Kaliumpermanganat werden in einer Mischung aus 80 ml Wasser und 20 ml 1N NaOH gelöst und 6 h bei Raumtemperatur gerührt. Anschließend wird mit 4 ml konzentrierter Schwefelsäure angesäuert und 10 min mit 40 ml gesättigter Natriumsulfit-Lösung gerührt. Es wird filtriert und das Filtrat 5 mal mit Methylenchlorid extrahiert. Die vereinigten organischen Extrakte werden getrocknet und vom Lösungsmittel befreit. Der in Ether aufgenommene Rückstand wird mit 1N NaOH extrahiert. Die wäßrige Phase wird mit 1N HCl auf pH 2 angesäuert und mit Methylenchlorid extrahiert. Nach der Trocknung verbleiben 0,54 g (51 %) (S)-Ketoprofen.

Enantiomerenüberschuß: 80 % e.e.

Beispiel 2

Oxidation von 2-(S)-(3-Methoxy-phenyl-methyl)-phenyl)-propansäure

Eine Lösung von 3,00 g (11,1 mmol) des Produktes von Beispiel 5 in 45 ml Tetrachlorkohlenstoff werden zusammen mit 2,2 g (12,2 mmol) N-Brom-Succinimid und einer Spatelspitze Dibenzoylperoxid am Rückfluß erhitzt. Nach 3,5 h wird die erkaltete Lösung filtriert, das Filtrat vom Lösungsmittel befreit und in Essigester gelöst. Die organische Phase wird dreimal mit 1N NaOH extrahiert. Die vereinigten, angesäuerten wäßrigen Phasen werden zweimal mit Essigester extrahiert. Die vereinigten organischen Phasen werden mit gesättigter Natriumchloridlösung und Magnesiumsulfat getrocknet und einrotiert. Ausbeute 2,59 g (91 %) (S)-Ketoprofen.
Enantiomerenüberschuß: 92 % e.e.

Beispiel 3

Oxidation von 2-(3-(Hydroxy-phenyl-methyl)-phenyl)-propansäure

Man gibt zu 1,00 g (3,9 mmol) Produkt des Beispiels 6 in 5 ml Methanol 6 ml einer 1M Lösung von Brom (6 mmol) in Methanol und läßt bei Raumtemperatur bis zur Abreaktion des Eduktes rühren (ca. 4h). Anschließend wird die Reaktionslösung mit Eiswasser und 0,948 g (6 mmol) Natriumthiosulfat behandelt und am Rotationsverdampfer eingeengt. Durch Extraktion des wäßrigen Rückstandes mit Essigester, Trocknung der organischen Phase und Säulenchromatographie an Kieselgel gewinnt man 0,694 mg (70 %) (S)-Ketoprofen.
Enantiomerenüberschuß: 84 % e.e.

Beispiel 4a

Asymmetrische Hydrierung von 2-(3-Benzyl-phenyl)-propensäure

In einen 100 ml Autoklaven wird eine Lösung von 0,440 g (1,64 mmol) 2-(3-Benzyl-phenyl)-propensäure (Produkt von Beispiel 7 oder 8) in 5 ml sauerstoffreien Methanol und 10 mg (0,012 mmol) Ru-((S)-BINAP-$O_2CCH_3)_2$) unter Schutzgas eingefüllt. Nach 48 h Reaktion unter 100 atm Wasserstoff wird das Lösungsmittel entfernt, der Rückstand mit Essigester aufgenommen und zur Entfernung des Katalysators über Kieselgel filtriert. Ausbeute 0,42 g (95 %).
Enantiomerenüberschuß: 80 % e.e.

$^1$H-NMR (250 MHz, $CDCl_3$) $\delta$ = (ppm) 1,42 (d, J=7 Hz; 3H, $CH_3$), 3,62 (q, J=7,5 Hz; 1H, $CHCH_3$), 3,90 (s; 2H, $CH_2$), 7,1-7,3 (m; 9H, $H_{aromat.}$).

Beispiel 4b (Ausführungsvariante)

Der Versuch wurde analog zu Beispiel 4a, aber mit 10 mg [(p-$(CH_3)_2CH$-$C_6H_4$-$CH_3$)Ru(S)-BINAP J]$^+$J$^-$ (0,0090 mmol) (EPA 366 390) durchgeführt.
Ausbeute: 0,425 g (96 %)
Enantiomerenüberschuß: 85 % e.e.

Beispiel 4c (Ausführungsvariante)

7,8 mg (0,011 mmol) (S)-BINAP, 3,57 mg (0,011 mmol) [(p-$(CH_3)_2CH$-$C_6H_4$-$CH_3$)$RuCl_2$] in 3 ml DMF wurden 10 min auf 100°C erhitzt und anschließend wurde das Lösungsmittel im Pumpenvakuum entfernt. Der Rückstand wurde als Katalysator analog dem Beispiel 4a verwendet.
Ausbeute: 0,42 g (95 %)
Enantiomerenüberschuß: 80 % e.e.

Beispiel 5

Asymmetrische Hydrierung von 2-(3-(Methoxy-phenyl-methyl)-phenyl)-propensäure

Das Produkt von Beispiel 10 wurde analog zu Beispiel 4 umgesetzt. Aus 0,44 g Edukt wurden 0,42 g (94 %) 2-(S)-(3-(Methoxy-phenyl-methyl)-phenyl)-propionsäure erhalten.

Die Enantiomerenanalytik erfolgte auf der Stufe des Ketoprofens (siehe Beispiel 2)

$^1$H-NMR (250 MHz, $CDCl_3$) $\delta$ = (ppm) 1,48 (2xd, J=7 Hz; 3H, $CH_3$), 3,38 (s; $OCH_3$), 3,72 (q, J=7,5 Hz; $CHCH_3$), 5,21 (s; 1H, $HCCH_3$), 7,1-7,4 (m; 9H, $H_{aromat.}$).

Beispiel 6

Asymmetrische Hydrierung von 2-(3-(Hydroxy-phenyl-methyl)-phenyl)-propensäure

Das Produkt von Beispiel 9 wurde analog zu Beispiel 4 umgesetzt. Aus 0,44 g Edukt wurden 0,42 g (94 %) 2-(S)-(3-(Hydroxy-phenyl-methyl)-phenyl)-propionsäure erhalten.

Die Enantiomerenanalytik erfolgte auf der Stufe des Ketoprofens (siehe Beispiel 3).

$^1$H-NMR (250 MHz, $CDCl_3$) $\delta$ = (ppm) 1,49 (d, J=7 Hz; 3H, $CH_3$), 3,73 (q, J=7,5 Hz; 1H, $CHCH_3$), 5,81 (s; 1H, HOCH), 7,1-7,4 (m; 9H, $H_{aromat.}$).

Beispiel 7

Herstellung von 2-(3-Benzyl-phenyl)-propensäure

136 g (0,531 mol) 2-Hydroxy-2-(3-benzyl-phenyl)-propansäure (Produkt des Beispiels 11) wird in einer Mischung aus 3 l Dioxan und 180 ml konzentrierter Schwefelsäure 12 h unter Rückfluß gekocht. Das auf ca. 500 ml eingeengte Gemisch wurde mit Wasser versetzt und dreimal mit Ether extrahiert. Nach Trocknung der organischen Phasen mit gesättigtem NaCl und Magnesiumsulfat bleibt ein braunes Öl, welches durch Filtration über 1 kg Kieselgel (Essigester/Cyclohexan 5:1) gereinigt wurde.
Ausbeute: 83 g (66 %).

$^1$H-NMR (250 MHz, $CDCl_3$) $\delta$ = (ppm) 3,99 (s; 2H, $ArCH_2Ar$), 5,95 (d; 1H, C=$CH_2$), 6,47 (d; 1H, C=$CH_2$), 7,1-7,5 (m; 9H, H-aromat.).

GC/MS/CI (Isobutan) m/z = 239 (100 %, $M^+$+1), 238 (80 %, $M^+$), 161 (90 %, $m^+$-$C_6H_5$), 91 (80 %, $CH_2Ph^+$).

Beispiel 8

Darstellung von 2-(3-Benzyl-phenyl)-propensäure

Eine Mischung von 3.48 g (13,80 mmol) 2-(3-Benzyl-phenyl)-propensäuremethylester (Produkt des Beispiels 14a) in 2,70 g Lithiumhydroxid, 25 ml Wasser, 27 ml Acetonitril und 27 ml Heptan wird über Nacht unter intensiver Rührung mit einem Flügelrührer behandelt. Danach wird die Acetonitril/Wasser-Phase abgetrennt und eingeengt. Der erhaltene Rückstand wird in Essigester aufgenommen und mit Natriumhydroxid-Lösung extrahiert. Der basische Extrakt wird mit Salzsäure angesäuert und mit Essigester extrahiert, getrocknet und eingeengt.

Ausbeute: 1,87 g (8.00 mmol) (57.0 % d.Th.)

Die spektroskopischen Daten sind mit denen der Verbindung aus Beispiel 7 identisch.

Beispiel 9

Darstellung von 2-(3-(Hydroxy-phenyl-methyl)-phenyl)-propensäure

Eine Mischung von 2.40 g (8.96 mmol) 2-(3-(Hydroxy-phenyl-methyl)-phenyl)-propensäuremethylester (Produkt des Beispiels 15a) in 1.76 g Lithiumhydroxid, 16 ml Wasser, 18 ml Acetonitril und 18 ml Heptan wird über Nacht unter intensiver Rührung mit einem Flügelrührer behandelt. Danach wird die Acetonitril/Wasser-Phase abgetrennt und eingeengt. Der erhaltene Rückstand wird in Essigester aufgenommen und mit Natriumhydroxid-Lösung extrahiert. Der basische Extrakt wird mit Salzsäure angesäuert und mit Essigester extrahiert, getrocknet und eingeengt.
Ausbeute: 1.87 g (7.36 mmol) (82.1 % d.Th.)

Beispiel 10

Darstellung von 2-(3-(Methoxyphenyl-methyl)-phenyl)-propensäure

Eine Mischung von 97.26 g (345.00 mmol) 2-(3-(Methoxy-phenyl-methyl)-phenyl)-propensäuremethylester (Produkt des Beispiels 16a) in 67.00 g Lithiumhydroxid, 620 ml Wasser, 670 ml Acetonitril und 670 ml Heptan wird für 3 Tage unter intensiver Rührung mit einem Flügelrührer behandelt. Danach wird die Acetonitril/Wasser-Phase abgetrennt und eingeengt. Der erhaltene Rückstand wird in Acetessigester aufgenommen und mit Natriumhydroxid-Lösung extrahiert. Der basische Extrakt wird mit Salzsäure angesäuert und mit Essigester extrahiert, getrocknet und eingeengt.

Ausbeute: 45.99 g (171.60 mmol) (49.7 % d.Th.)

$^1$H-NMR: ($CDCl_3$, 200 MHz) $\delta$ = 3,38 (s, 3H; $-OCH_3$), 5,26 (s, 1H; -CH), 5,96 (s, 1H; $=CH_2$), 6,48 (s, 1H; $=CH_2$), 7,15-7,46 (m, 9H; aromat. H), 11,47 (s, br, 1H; -COOH)

MS-CI: (Isobutan, 70 eV) m/e = 269 (10 %, $M+H^+$), 237 (100 %), 211 (65 %), 191 (10 %), 121 (10 %)

Beispiel 11

Herstellung von 2-Hydroxy-2-(3-benzyl-phenyl)-propansäure durch das Pyruvatverfahren

Zu 1,62 g (67,5 mmol) Magnesium in 90 ml THF tropft man 15 g (60,7 mmol) 3-Benzyl-brombenzol (J. Chem. Soc. 1961, 1405) in 30 ml THF so zu, daß die Mischung lebhaft siedet und kocht noch weitere 2 h. Die erkaltete Lösung wird zu einer Suspension von 6,68 g (71 mmol) Natriumpyruvat bei 0°C in 20 ml THF zugetropft und es wird noch 1 h bei dieser Temperatur und 2 h unter Rückfluß gerührt. Nach Zugabe von 1N HCl wird das Gemisch weitgehend eingeengt und Essigester zugegeben. Die organische Phase wird abgetrennt und zweimal mit 1N NaOH extrahiert. Die vereinigten wäßrigen Lösungen werden mit konzentriertem HCl auf pH 2 eingestellt und zweimal mit Essigester extrahiert. Nach Trocknung der organischen Phase mit gesättigter Natriumchloridlösung und Magnesiumsulfat wird das Lösungsmittel abdestilliert und es verbleiben 12,5 g (81 %) des Titelproduktes.

$^1$H-NMR (250 MHz, $CDCl_3$) $\delta$ = (ppm) 1,81 (s; 3H, $CCH_3$), 40,1 (s; 3H, $ArCH_2Ar$), 7,1-7,5 (m; 9H, $H_{aromat.}$).

MS/CI (Isobutan) m/z = 257 (5 %, $M^+$+1), 239 (100 %, 257-$H_2O$), 211 (60 %, 257-CO-$H_2O$).

Beispiel 12a

Herstellung von 2-Hydroxy-2-(3-(methoxy-phenyl-methyl)-phenyl)-propansäure durch das Pyruvatverfahren

Zu 3,14 g (0,131 mmol) Magnesium in 200 ml THF tropft man 30 g (108 mmol) 3-Brom-diphenyl-methoxymethan (Produkt des Beispiels 16d) in 60 ml THF so zu, daß die Mischung lebhaft siedet und kocht noch weitere 2 h. Die erkaltete Lösung wird zu einer Suspension von 21 g (190 mmol) Natriumpyruvat bei 0°C zugetropft und es wird noch 24 h bei 50°C gerührt. Nach Zugabe von Ammoniumchloridlösung wird das Gemisch weitgehend eingeengt und Essigester zugegeben. Die organische Phase wird abgetrennt und zweimal mit 1N NaOH extrahiert. Die vereinigten wäßrigen Lösungen werden mit konzentriertem HCl auf pH 2 eingestellt und zweimal mit Essigester extrahiert. Nach Trocknung der organischen Phase mit gesättigter Natriumchlorid-Lösung und Magnesiumsulfat wird das Lösungsmittel abdestilliert und es verbleiben 27,5 g (88,7 %) des Titelproduktes.

$^1$H-NMR (250 MHz, $CDCl_3$) $\delta$ = (ppm) 1,77 (s; 3H, C-$CH_3$), 3,36 (s; 3H, $OCH_3$), 5,26 (s; 1H, $ArCH_2Ar$), 7,2-7,7 (m; 9H, H-Ar).

MS/CI (Isobutan) m/z = 269 (50 %, $M^+$+1-$H_2O$), 255 (100 %, $M^+$+1-$OCH_3$), 241 (40 %, 255-CO).

Beispiel 12b (Alternativverfahren)

Herstellung von 2-Hydroxy-2-(3-(methoxy-phenyl-methyl)-phenyl)-propansäure durch das Pyruvatverfahren/$ZnCl_2$

Zu 0,290 g (11,9 mmol) Magnesium in 15 ml THF tropft man 2,77 g (10,0 mmol 3-Brom-diphenyl-methoxymethan (Produkt des Beispiels 16d) in 60 ml THF so zu, daß die Mischung lebhaft siedet und kocht noch weitere 2 h. Die erkaltete Lösung wird abdekantiert und bei Raumtemperatur mit 0,68 g (4,99 mmol) trockenem Zink(II)chlorid gerührt.

Das Reaktionsgemisch wird zu einer Suspension von 1,1 g (10,0 mmol) Natriumpyruvat in 200 ml THF bei 0°C zugetropft und es wird noch 24 h bei 50°C gerührt. Nach Zugabe von Ammoniumchloridlösung wird das Gemisch weitgehend eingeengt und Essigester zugegeben. Die organische Phase wird abgetrennt und zweimal mit 1N NaOH extrahiert. Die vereinigten wäßrigen Lösungen werden mit konzentriertem HCl auf pH 2 eingestellt und zweimal mit Essigester extrahiert. Nach Trocknung der organischen Phase mit gesättigter Natriumchlorid-Lösung und Magnesiumsulfat wird das Lösungsmittel abdestilliert und es verbleiben 1,99 g (70 %) des Titelproduktes.

Beispiel 13a

Herstellung von 2-Hydroxy-2-(3-(hydroxy-phenyl-methyl)-phenyl)-propansäure durch das Pyruvatverfahren

Zu 0,228 g (9,5 mmol) Magnesium in 30 ml THF tropft man 3,35 g (10 mmol) 3-Brom-diphenyl-trimethylsiloxymethan in 10 ml Tetrahydrofuran so zu, daß die Mischung lebhaft siedet und kocht noch weitere 2 h. Die erkaltete Lösung wird zu einer Suspension von 1,1 g (10 mmol) Natriumpyruvat bei 40°C gegeben, es wird 1 h bei dieser Temperatur und 24 h unter Rückfluß gerührt.

Nach Zugabe von 15 ml 1N HCl wird 1 h gerührt und das Gemisch weitgehend eingeengt und Essigester zugegeben. Die organische Phase wird abgetrennt und zweimal mit 1n NaOH extrahiert. Die vereinigten wäßrigen Lösungen werden mit konzentriertem HCl auf pH 2 eingestellt und zweimal mit Essigester extrahiert. Nach Trocknung der organischen Phase mit gesättigter Natriumchlorid-Lösung und Magnesiumsulfat wird das Lösungsmittel abdestilliert und der Rückstand an Kieselgel chromatographiert. Ausbeute 1,5 g (55 %) des Titelproduktes.

$^1$H-NMR (250 MHz, $CDCl_3$) $\delta$ = (ppm) 1,72 (s; 3H, $CCH_3$), 5,79 (s; 1H, HOCH), 7,2-7,7 (m; 9H, H-aromat.).

Beispiel 13b

Darstellung von 3-Brom-diphenyl-trimethylsiloxymethan

Eine Lösung von 50 g (0.19 mol) 3-Brom-diphenylmethanol, (Synth. Commun., 12, 881 (1982)), 26.8 g (0.25 mol) Trimethylsilylchlorid, 26.9 g Triethylamin, 1 g Dimethylaminopyridin in 600 ml Methylenchlorid wird 48 h bei RT gerührt, mit 1 % HCl, 1 % $NaHCO_3$ und mit ges. NaCl-Lösung extrahiert und mit $MgSO_4$ getrocknet. Nach dem Entfernen des Lösungsmittels wird durch fraktionierte Kieselgelfiltration (Essigester in Cyclohexan, 0-3 %) gereinigt. Ausbeute: 54 g (85 %).

$^1$H-NMR ($CDCl_3$, 250 MHz) $\delta$ = (ppm) 0.10 (s, 9H, $(CH_3)_3C$), 5.7 (S, 1H, $Ar_2CH$), 7,1-7,5 (m, 9H)

GC/MS/EI (70 eV) m/z = 336/334 (20 % $M^+$+1), 319/321 (30 %, $M^+$+1-$CH_3$), 255 (40 %, $M^+$-Br), 243/245 (80 %, $PhCHC_6H_4Br^+$), 165 (100 %, $C_6H_5CC_6H_4^+$), 73 (90 %, $Si(CH_3)_3^+$)

Beispiel 14a

Darstellung von 2-(3-Benzyl-phenyl)-propensäuremethylester

Eine Lösung von 2,25 g (11,80 mmol) 3-Ethinyl-diphenylmethan (Produkt der Beispiele 14b oder 14d), 0,71 g Natriumiodid, 0,224 g konzentrierte Salzsäure und 0,13 g Hydrochinon in 15 ml Methanol wird in einem 0,1 l VA-Autoklaven für 24 Stunden bei 60°C mit 20 bar Kohlenmonoxid beaufschlagt. Nach Entspannen des Autoklaven wird zur Trockne eingeengt und der Rückstand durch Chromatographie an Kieselgel gereinigt.

Ausbeute: 0,48 g (1,92 mmol) (16,3 % d.Th.).

MS-CI: 70 eV, Isobutan) m/e = 253 (100 %, M+$H^+$), 221 (6 %), 175 (8 %), 91 (6 %),
$^1$H-NMR: ($CDCl_3$, 200 MHz) $\delta$ = 3,74 (s, 3H; -$OCH_3$), 3,99 (s, 2H; -$CH_2$-), 5,85 (s, 1H; =$CH_2$), 6,33 (s, 1H; =$CH_2$), 6,85-7,35 (m, 9H; arom. H)

Neben dem Produkt wurden 1,10 g (5,76 mmol) (48,9 % d.Th.) des Eduktes reisoliert.

Beispiel 14b

Darstellung von 3-Ethinyl-diphenylmethan

Eine Lösung von 4,00 g (15,00 mmol) 3-(Trimethylsilylethinyl)-diphenylmethan in 200 ml Ethanol wird mit 0,97 g Kaliumfluorid versetzt und über Nacht bei Raumtemperatur gerührt. Es wird abgesaugt, eingeengt und durch Chromatographie an Kieselgel (Cyclohexan : Essigester 50:1 v:v) gereinigt.

Ausbeute: 2,25 g (11,70 mmol) (78,0 % d.Th.).

MS-CI: (70 eV, Isobutan) m/e = 193 (100 %, $M+H^+$), 157 (6 %), 115 (35 %), 91 (100 %)
$^1$H-NMR: ($CDCl_3$, 200 MHz) $\delta$ = 3,03 (s, 1H; =C-H), 3,96 (s, 2H; $-CH_2$), 7,10-7,40 (m, 9H; arom. H)

Beispiel 14c

Darstellung von 3-(Trimethylsilylethinyl)-diphenylmethan

Zu einer Lösung von 3,70 g (15,00 mmol) 3-Bromdiphenylmethan (J. Chem. Soc. 1961, 1405), 0,119 g (1,50 mmol) Palladium-bis-triphenylphosphin-dichlorid, 0,158 g (24,00 mmol) Triphenylphosphin, 0,029 g (0,20 mmol) Kupfer(I)iodid in 20 ml wasserfreiem Triethylamin wird unter Schutzgas mit 2,22 g (22,50 mmol) Trimethylsilylacetylen versetzt und über Nacht auf Rückfluß erhitzt, wobei der Intensivkühler mit einem Kühlaggregat auf -8°C temperiert wird. Man verdünnt mit Methylenchlorid, saugt von den ausgefallenen Salzen ab, wäscht zweimal mit Wasser, trocknet und engt ein. Das Rohprodukt wird durch Chromatographie über Kieselgel (Essigester : Cyclohexan 1:20 v:v) gereinigt.

Ausbeute: 4,05 g (15,30 mmol) (100 % d.Th.).

MS-EI: (70 eV) m/e = 264 (26 %, $M^+$), 249 (100 %), 124 (6 %), 91 (8 %)
$^1$H-NMR: ($CDCl_3$, 200 MHz) $\delta$ = 0,75 (s, 9H; $-Si-(CH_3)_3-$), 3,96 (s, 2H; $-CH_2-$), 7,10-7,40 (m, 9H; arom. H)

Beispiel 14d

Darstellung von 3-Ethinyldiphenylmethan aus 3-Hydroxy-3-methyl-butinyl-diphenylmethan

Eine Lösung von 2.50 g (9.54 mmol) 3-Hydroxy-3-methyl-butinyl-diphenylmethan in 20 ml Toluol wird mit 0,06 g Natriumhydrid (80 proz. in Weißöl) versetzt und über Nacht erhitzt, so daß nur wenig Toluol abdestillierte. Anschließend wurde abfiltriert, in Methylenchlorid aufgenommen und mit Salzsäure, Natriumcarbonat und Wasser gewaschen. Das nach der Trocknung und dem Einengen erhaltenen Rohprodukt wurde durch Chromatographie an Kieselgel im System Essigester/Cyclohexan (1/20 v/v) gereinigt.

Ausbeute: 1.54 g (8.02 mmol) (84.1 % d.Th.)
Spektroskopische Daten siehe Beispiel 14b

Beispiel 14e

Darstellung von 3-(3-Hydroxy-3-methyl-butinyl)-diphenylmethan

Zu einer Lösung von 6.00 g (24.30 mmol) 3-Bromdiphenylmethan (J. Chem. Soc. 161, 1405), 0.170 g (0.24 mmol) Palladium-bis-triphenylphosphin-dichlorid, 0.25 g (9.72 mmol) Triphenylphosphin, 0,046 g (0.24 mmol) Kupfer(I)iodid in 25 ml wasserfreiem Triethylamin wird unter Stickstoff bei 95°C mit 3.06 g (36.45 mmol) 2-Methyl-3-butin-2-ol versetzt und über Nacht auf Rückfluß erhitzt. Man verdünnt mit Methylenchlorid, saugt von den ausgefallenen Salzen ab, wäscht zweimal mit Wasser, trocknet und engt ein. Das Rohprodukt wird durch Chromatographie über Kieselgel gereinigt.

Ausbeute: 5.19 g (20.76 mmol) (85.4 % d.Th.)

MS-CI: (Isobutan, 70 eV) m/e = 251 (8 %, $M+H^+$), 250 (18 %, $M^+$), 233 (100 %), 193 (12 %), 173 (10 %), 91 (21 %)

Beispiel 15a

Darstellung von 2-(3-(Methoxy-phenyl-methyl)-phenyl-propionsäuremethylester und 2-(3-(Hydroxy-phenyl-methyl)-phenyl-propionsäuremethylester

Eine Lösung von 22.50 g (109.00 mmol) 3-Ethinyl-diphenylmethanol (Produkt der Beispiele 15b oder 15d), 6.60 g Natriumiodid, 2.12 g konz. Salzsäure und 1.19 g Hydrochinon in 132 ml Methanol wird in einem 0,3 l VA-Autoklaven für

24 Stunden bei 60°C mit 20 bar Kohlenmonoxid beaufschlagt. Nach Entspannen des Autoklaven wird zur Trockne eingeengt und der Rückstand durch Chromatographie an Kieselgel (Essigester:Cyclohexan 1:50 -> 1:1 v:v) gereinigt.

2-(3-(Methoxy-phenyl-methyl)-phenyl)-propionsäuremethylester

Ausbeute: 3.22 g (11.41 mmol) (10.5 % d. Th.)

$^1$H-NMR: ($CDCl_3$, 200 MHz) $\delta$ = 3,77 (s, 3H; $-OCH_3$), 3,93 (s, 3H; $-OCH_3$), 5,25 (s, 1H; $-CH-OCH_3$), 7,20 bis 7,57 (m, 9H; arom. H)

MS-EI: (EI, 70 eV) m/e = 282 (28 %, $M^+$), 251 (100 %), 250 (80 %), 222 (20 %), 205 (82 %), 191 (100 %), 189 (52 %), 178 (12 %), 165 (24 %), 121 (85 %), 105 (44 %).

2-(3-(Hydroxy-phenyl-methyl)-phenyl)-propionsäuremethylester

Ausbeute: 2.50 g (9.33 mmol) (8.6 % d.Th.)

MS-EI: (EI, 70 eV) m/e = 268 (28 %, $M^+$), 250 (12 %), 236 (22 %), 208 (80 %), 191 (26 %), 189 (87 %) 163 (84 %), 131 (78 %) 105 (96 %), 103 (100 %).

Neben den Produkten wurden 2.80 g (13.46 mmol) (12.3 % d.Th.) des Eduktes reisoliert und 2,99 g 3-Bromdiphenylmethoxymethan isoliert.

Beispiel 15b

Darstellung von 3-Ethinyl-diphenylmethanol

Eine Lösung von 6.30 g (22.00 mmol) 3-(Trimethylsilylethinyl)-diphenylmethanol in 50 ml Ethanol wird mit 1.44 g Kaliumfluorid versetzt und über Nacht bei Raumtemperatur gerührt. Es wird abgesaugt, eingeengt und durch Chromatographie an Kieselgel (Cyclohexan:Essigester 50:1 v:v) gereinigt.
Ausbeute: 3.45 g (15.90 mmol) (72.0 % d.Th.)

MS-EI: (EI, 70 eV) m/e = 208 (84 %, $M^+$), 189 (17 %), 178 (12 %), 129 (65 %), 105 (100 %), 103 (28 %)

Beispiel 15c

Darstellung von 3-(Trimethylsilylethinyl)-diphenylmethanol

Zur einer Lösung von 7.89 g (30.00 mmol) 3-Bromdiphenylmethanol (Synth. Commun. 12, 881 (1982)), 0.237 g (0.30 mmol) Palladium-bis-triphenylphosphindichlorid, 0.315 g (1.20 mmol) Triphenylphosphin, 0,057 g (0.30 mmol) Kupfer(I)-iodid in 30 ml wasserfreiem Triethylamin wird unter Stickstoff mit 4.49 g (45.00 mmol) Trimethylsilylacetylen versetzt und über Nacht auf Rückfluß erhitzt, wobei der Intensivkühler mit einem Kühlaggregat auf -8°C temperiert wird. Man verdünnt mit Methylenchlorid, saugt von den ausgefallenen Salzen ab, wäscht zweimal mit Wasser, trocknet und engt ein. Das Rohprodukt wird durch Chromatographie über Kieselgel (Essigester:Cyclohexan 1:20 v:v) gereinigt.
Ausbeute: 6.35 g (22.00 mmol) (73.0 % d. Th.)

MS-CI: (70 eV, Isobutan) m/e = 279 (10 %, $M+H^+$), 211 (5 %), 201 (10 %), 183 (5 %)

$^1$H-NMR: ($CDCl_3$, 200 MHz) $\delta$ = 0,25 (s, 9H; $-Si(CH_3)_3$), 2,28 (s, 1H; -CH-OH), 5,77 (s, 1H; -CH-OH), 7,20-7,57 (m, 9H; arom. H)

Beispiel 15d

Darstellung von 3-Ethinyldiphenylmethanol aus 3-(3-Hydroxy-3-methyl-butinyl)-diphenylmethan

Eine Lösung von 10.00 g (35.97 mmol) in 3-(3-Hydroxy-3-methyl-butinyl)-diphenylmethanol in Toluol wird mit 0,36 g Natriumhydrid 60 proz. in Weißöl versetzt und über Nacht erhitzt, so daß nur wenig Toluol abdestilliert. Anschließend wurde abfiltriert, in Methylenchlorid aufgenommen und mit Natriumcarbonat und Wasser gewaschen. Das nach der Trocknung und dem Einengen erhaltene Rohprodukt wurde durch Chromatographie an Kieselgel (Essigester:Cyclohexan 1:10 v:v) gereinigt.
Ausbeute: 0.78 g (3.59 mmol) (10 % d.Th.)

Die spektroskopischen Daten stimmen mit denen der Verbindung aus Beispiel 15b überein.

Beispiel 15e

Darstellung von 3-(3-Hydroxy-3-methyl-butinyl)-diphenylmethanol

Zu einer Lösung von 18.00 g (68.40 mmol) 3-Bromdiphenylmethanol (Synth. Commun. 12, 881 (1982)), 0.48 g (0,68 mmol) Palladium-bis-triphenylphosphin-dichlorid, 0,71 g (2.70 mmol) Triphenylphosphin, 0,13 g (0.68 mmol) Kupfer-(I)-iodid in 20 ml wasserfreiem Triethylamin wird unter Schutzgas bei 95°C mit 8.62 g (102.60 mmol) 2-Methyl-3-butin-2-ol versetzt und über Nacht auf Rückfluß erhitzt. Man verdünnt mit Methylenchlorid, saugt von den ausgefallenen Salzen ab, wäscht zweimal mit Wasser, trocknet und engt ein. Das Rohprodukt wird durch Chromatographie über Kieselgel (Laufmittel: Essigester:Cyclohexan 1:3 v:v) gereinigt.
Ausbeute: 20.10 g (15.4 mmol) (quant.)

MS-CI: (Isobutan, 70 eV) m/e = 266 (12 %, $M+H^+$), 249 (100 %), 191 (33 %)

Beispiel 16a

Darstellung von 2-(3-(Methoxy-phenyl-methyl)-phenyl)-propensäuremethylester

Eine Lösung von 104.80 g (0.472 mol) 3-Ethinyl-diphenylmethoxymethan, 28.60 g Natriumiodid, 9.18 g konz. Salzsäure und 5.15 g Hydrochinon in 570 ml Methanol und 250 ml Toluol wird in einem 1.3 l VA-Autoklaven für 12 Stunden bei 60°C mit 20 bar Kohlenmonoxid beaufschlagt. Nach Entspannen des Autoklaven wird zur Trockne eingeengt und der Rückstand durch Chromatographie an Kieselgel (Essigester:Cyclohexan 1:50 -> v:v) gereinigt.
Ausbeute: 27.60 g (97.87 mmol) (20.7 % d.Th.)
Die spektroskopischen Daten stimmen mit denen der Verbindung aus Beispiel 15a überein.
Neben dem Produkt wurden 25.32 g (114.05 mmol) (24.2 % d.Th.) des Eduktes reisoliert.

Beispiel 16b

Darstellung von 3-Ethinyl-diphenylmethoxymethan aus 3-(3-Hydroxy-3-methyl-butinyl)-diphenylmethoxymethan

Eine Suspension von 225.20 g (0.80 mol) 3-(3-Hydroxy-3-methyl-butinyl)-diphenylmethoxymethan und 32.80 g gepulvertem Natriumhydroxid in 3000 ml wasserfreiem Toluol wird unter Stickstoff für 16 Stunden auf Rückfluß erhitzt. Danach wird das gepulverte Natriumhydroxid abgesaugt und man wäscht zweimal mit Wasser, trocknet und engt ein.
Das Rohprodukt wird durch Chromatographie über Kieselgel (Laufmittel: Essigester:Cyclohexan 1:10 v:v) gereinigt.
Ausbeute: 439.68 g (1.57 mol) (83 % d.Th.)

$^1$H-NMR: ($CDCl_3$, 200 MHz) $\delta$ = 2,34 (s, 1H; =C-H), 3,36 (s, 3H; $-OCH_3$), 5,19 (s, 1H; -CH-), 7,10 bis 7,45 (m, 9H; arom. H)

Beispiel 16c

Darstellung von 3-(3-Hydroxy-3-methyl-butinyl)-diphenylmethoxymethan

Zu einer Lösung von 524.65 g (1.90 mol) 3-Brom-diphenylmethoxymethan, 13.30 g (19.00 mmol) Palladium-bis-triphenylphosphindichlorid, 19.76 g (76.00 mmol) Triphenylphosphin, 3.59 g (19.00 mmol) Kupfer-(I)-iodid in 20 ml wasserfreiem Triethylamin wird unter Stickstoff bei 95°C mit 237.17 g (2.85 mol) 2-Methyl-3-butin-2-ol versetzt und über Nacht auf Rückfluß erhitzt. Man verdünnt mit Methylenchlorid, saugt von den ausgefallenen Salzen ab, wäscht zweimal mit Wasser, trocknet und engt ein. Das Rohprodukt wird durch Chromatographie über Kieselgel (Laufmittel: Essigester:Cyclohexan 1:10 v:v) gereinigt.
Ausbeute: 439.68 g (1.57 mol) (83 % d.Th.)

$^1$H-NMR: ($CDCl_3$, 200 MHz) $\delta$ = 1,40 (s, 6H; $-CH_3$), 2,10 (s, br, 1H; -OH), 3,37 (s, 3H; $-OCH_3$), 5,19 (s, 1H, -CH), 7,20-7,46 (m, 9H; aromat. H)
MS-CI: (Isobutan, 70 eV) m/e = 281 (6 %, $M+H^+$), 280 (18 %, $M^+$), 263 (100 %), 249 (64 %), 191 (94 %), 121 (40 %)

Beispiel 16d

Darstellung von 3-Brom-diphenylmethoxymethan

Eine Lösung von 272.00 g (1.03 mol) 3-Brom-diphenylmethanol (J. Chem. Soc., 1961, 1405) in 2500 ml wasserfreiem Tetrahydrofuran wird bei ca. 15°C portionsweise mit insgesamt 42.00 g Natriumhydrid 80 proz. in Weißöl versetzt. Nach beendeter Wasserstoffentwicklung läßt man noch eine Stunde bei Raumtemperatur nachrühren, danach hält man auf 50-60°C und tropft 98.0 ml Methyliodid zu. Nach beendeter Zugabe wird noch 4 Stunden auf Rückfluß erhitzt. Es wird dann unter Kühlung mit 2N Salzsäure versetzt, mit 1250 ml Essigester zugesetzt und zweimal mit Wasser extrahiert. Das nach der Trocknung und dem Einengen erhaltene Rohprodukt wird destilliert. Man erhält ein leicht gelbliches Öl.

Ausbeute: 165.65 g (0.59 mol) (58.1 % d.Th.)

$^1$H-NMR: ($CDCl_3$, 200 MHz) $\delta$ = 3,36 (s, 3H; $-OCH_3$), 5,18 (s, 1H; -CH-), 7,10-7,55 (m, 9H; aromat. H)
MS-EI: (EI, 70 eV) m/e = 222 (82 %, M+), 207 (26 %), 191 (100 %) 189 (58 %), 145 (50 %), 129 (24 %) 121 (70 %), 115 (8 %), 105 (28 %), 101 (14 %)

**Patentansprüche**

**Patentansprüche für folgende Vertragsstaaten : AT, BE, CH, DE, DK, FR, GB, IT, LI, LU, MC, NL, PT, SE**

**1.** (2)-Arylpropensäuren der allgemeinen Formel (II)

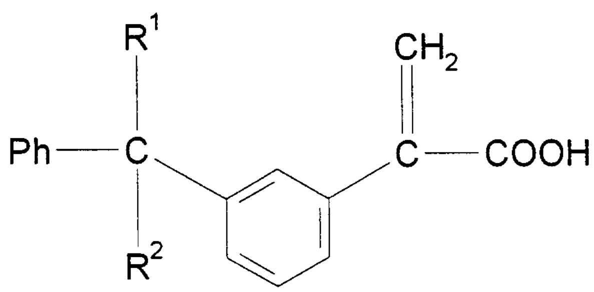

(II),

in welcher

$R^1$ und $R^2$ gleich oder verschieden sind und jeweils für Wasserstoff, Hydroxy, Alkoxy mit bis zu 10 C-Atomen, Cycloalkoxy mit 5 bis 10 C-Atomen, Aralkoxy mit 7 bis 12 C-Atomen oder Aryloxy mit 6 bis 10 C-Atomen, der gegebenenfalls substituiert ist durch Alkyl oder Alkoxy mit jeweils 1 bis 5 C-Atomen, stehen

oder
für die Gruppe $O_2CR^3$ stehen, wobei $R^3$ für Wasserstoff, Alkyl mit bis zu 10 C-Atomen, Cycloalkyl mit 5 bis 10 C-Atomen, Aralkyl mit 7 bis 12 C-Atomen oder Aryl mit 6 bis 10 C-Atomen, gegebenenfalls substituiert mit Alkyl oder Alkoxy mit jeweils 1 bis 5 C-Atomen steht,
oder
für die Gruppe $OSiR^4R^5R^6$ stehen, wobei $R^4$, $R^5$ und $R^6$ unabhängig voneinander jeweils die für $R^3$ angegebene Bedeutung haben
oder
für die Gruppe $NR^7R^8$ stehen, wobei $R^7$ und $R^8$ unabhängig voneinander die für $R^3$ angegebene Bedeutung haben
oder
für eine 2-Tetrahydropyrylgruppe steht
oder

$R^1$ und $R^2$ gemeinsam für eine Gruppierung der Struktur

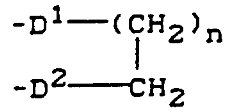

stehen, worin

$D^1$ und $D^2$ unabhängig voneinander jeweils Sauerstoff, Schwefel oder die Gruppe $NR^3$ bedeuten, wobei $R^3$ die oben angegebene Bedeutung hat und

n für 1, 2 oder 3 steht.

2. Verbindungen der allgemeinen Formel (II) gemäß Anspruch 1, in welcher

$R^1$ und $R^2$ gleich oder verschieden sind und jeweils für Wasserstoff, Hydroxyl, Alkoxy mit 1 bis 4 C-Atomen, Benzyloxy, Acetyloxy, Benzoyloxy, Tri(alkyl)silyl mit jeweils 1 bis 4 C-Atomen in den Alkylresten, Dialkylamino mit jeweils 1 bis 4 C-Atomen in den Alkylresten oder für 2-Tetrahydropyryl steht

oder

$R^1$ und $R^2$ zusammen für die Gruppe $-O(CH_2)_n-CH_2-D^2$-stehen, wobei n für 1, 2 oder 3 steht und $D^2$ für Sauerstoff oder Alkylamin mit 1 bis 4 C-Atomen steht.

3. Verfahren zur Herstellung von Verbindungen der allgemeinen Formel (II) gemäß Anspruch 1, dadurch gekennzeichnet, daß man

A) Verbindungen der allgemeinen Formel (III)

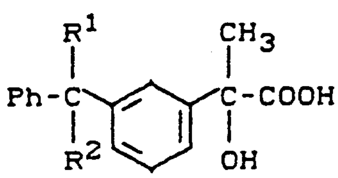

(III)

in welcher

$R^1$ und $R^2$ die oben angegebene Bedeutung haben,

nach üblichen Methoden dehydratisiert
oder

B) Verbindungen der Formel (IV)

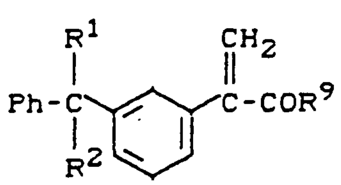

(IV)

in welcher

$R^1$ und $R^2$ die oben angegebene Bedeutung haben und
$R^9$ für Alkoxy mit 1 bis 10 C-Atomen, Cycloalkoxy mit 5 bis 10 C-Atomen, Aralkoxy mit 7 bis 12 C-Atomen oder Aryloxy mit 6 bis 10 C-Atomen, das gegebenenfalls durch Alkyl oder Alkoxy mit jeweils 1 bis 5 C-Atomen substituiert ist, steht,

nach üblichen Methoden hydrolysiert.

**4.** Verwendung von Verbindungen der allgemeinen Formel (II) gemäß Anspruch 1 zur Herstellung von (S)-Ketoprofen, dadurch gekennzeichnet, daß man die Verbindungen der allgemeinen Formel (II) in Gegenwart eines Katalysators der aus einem chiralen Übergangs-Metallkomplex besteht und gegebenenfalls in Gegenwart von inerten organischen Lösungsmitteln zu Verbindungen der allgemeinen Formel (I)

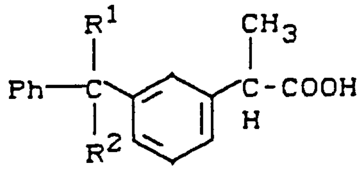

(I)

in welcher

$R^1$ und $R^2$ die oben angegebene Bedeutung haben, aber nicht gleichzeitig für Wasserstoff stehen,

hydriert und anschließend die Gruppe $CR^1R^2$ nach üblichen Methoden in eine Ketogruppe umwandelt.

**5.** Verbindungen der allgemeinen Formel (III)

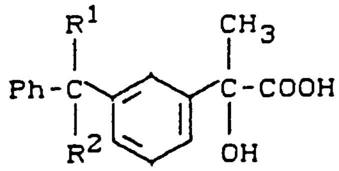

(III)

in welcher

$R^1$ und $R^2$ die im Anspruch 1 angegebene Bedeutung haben.

**6.** Verbindungen der allgemeinen Formel (IV)

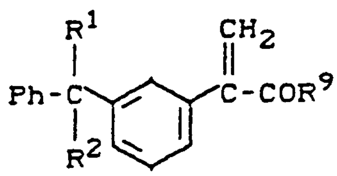

(IV)

in welcher

$R^1$ und $R^2$ die im Anspruch 1 angegebene Bedeutung haben und

$R^9$ für Alkoxy mit 1 bis 10 C-Atomen, Cycloalkoxy mit 5 bis 10 C-Atomen, Aralkoxy mit 7 bis 12 C-Atomen oder Aryloxy mit 6 bis 10 C-Atomen, das gegebenenfalls durch Alkyl oder Alkoxy mit jeweils 1 bis 5 C-Atomen substituiert ist, steht.

**7.** Verfahren zur Herstellung von Verbindungen der allgemeinen Formel (IV), dadurch gekennzeichnet, daß man Verbindungen der allgemeinen Formel (V)

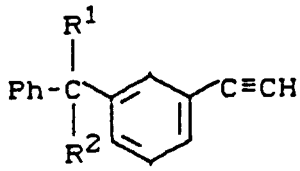

(V)

in welcher

$R^1$ und $R^2$ die im Anspruch 1 angegebene Bedeutung haben,

in Gegenwart von Palladium auf Aktivkohle als Katalysator und Kohlenmonoxid bei Drucken zwischen 1 und 200 bar mit einem Alkohol unter Zugabe von Mineralsäure, gegebenenfalls in Anwesenheit eines inerten aprotischen Lösungsmittels bei Temperaturen zwischen 25 und 160°C umsetzt.

**8.** Verfahren zur Herstellung von Verbindungen der allgemeinen Formel (III), dadurch gekennzeichnet, daß man Grignard-Verbindungen der Formel (VI)

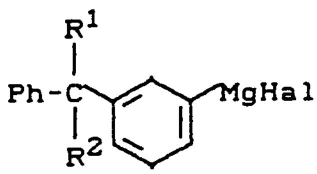

(VI)

in welcher

$R^1$ und $R^2$ die im Anspruch 1 angegebene Bedeutung haben und

Hal für Fluor, Jod, Chlor oder Brom steht,

mit einem Metall-pyruvat der allgemeinen Formel (VII)

$$Me^{\oplus}\ {}^{\ominus}OCC\text{-}CO\text{-}CH_3 \qquad (VII)$$

in welcher

Me für Alkali, Erdalkali (ein halbes Äquivalent) oder MgBr steht,

in Gegenwart von organischen Lösungsmitteln bei Temperaturen zwischen -10°C und +120°C umsetzt.

**Patentansprüche für folgende Vertragsstaaten : ES, GR**

**1.** Verfahren zur Herstellung von Verbindungen der allgemeinen Formel (II)

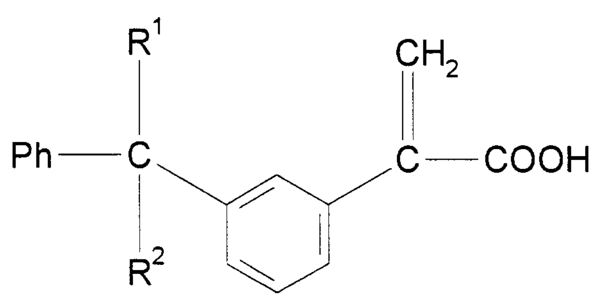

(II)

in welcher

$R^1$ und $R^2$ gleich oder verschieden sind und jeweils für Wasserstoff, Hydroxy, Alkoxy mit bis zu 10 C-Atomen, Cycloalkoxy mit 5 bis 10 C-Atomen, Aralkoxy mit 7 bis 12 C-Atomen oder Aryloxy mit 6 bis 10 C-Atomen, der gegebenenfalls substituiert ist durch Alkyl oder Alkoxy mit jeweils 1 bis 5 C-Atomen, stehen

oder
für die Gruppe $O_2CR^3$ stehen, wobei $R^3$ für Wasserstoff, Alkyl mit bis zu 10 C-Atomen, Cycloalkyl mit 5 bis 10 C-Atomen, Aralkyl mit 7 bis 12 C-Atomen oder Aryl mit 6 bis 10 C-Atomen, gegebenenfalls substituiert mit Alkyl oder Alkoxy mit jeweils 1 bis 5 C-Atomen steht,
oder
für die Gruppe $OSiR^4R^5R^6$ stehen, wobei $R^4$, $R^5$ und $R^6$ unabhängig voneinander jeweils die für $R^3$ angegebene Bedeutung haben
oder
für die Gruppe $NR^7R^8$ stehen, wobei $R^7$ und $R^8$ unabhängig voneinander die für $R^3$ angegebene Bedeutung haben
oder
für eine 2-Tetrahydropyrylgruppe steht
oder

$R^1$ und $R^2$ gemeinsam für eine Gruppierung der Struktur

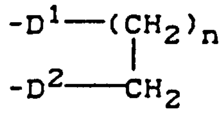

stehen, worin

$D^1$ und $D^2$ unabhängig voneinander jeweils Sauerstoff, Schwefel oder die Gruppe $NR^3$ bedeuten, wobei $R^3$ die oben angegebene Bedeutung hat und
n für 1, 2 oder 3 steht,

dadurch gekennzeichnet, daß man

A) Verbindungen der allgemeinen Formel (III)

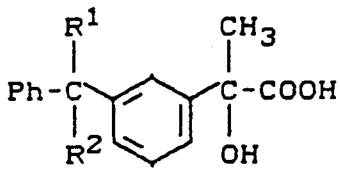

(III)

in welcher

$R^1$ und $R^2$ die oben angegebene Bedeutung haben,

nach üblichen Methoden dehydratisiert
oder

B) Verbindungen der Formel (IV)

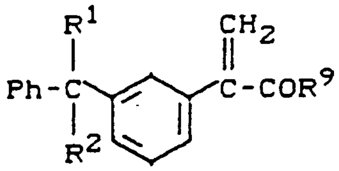

(IV)

in welcher

$R^1$ und $R^2$ die oben angegebene Bedeutung haben und
$R^9$ für Alkoxy mit 1 bis 10 C-Atomen, Cycloalkoxy mit 5 bis 10 C-Atomen, Aralkoxy mit 7 bis 12 C-Atomen oder Aryloxy mit 6 bis 10 C-Atomen, das gegebenenfalls durch Alkyl oder Alkoxy mit jeweils 1 bis 5 C-Atomen substituiert ist, steht,

nach üblichen Methoden hydrolysiert.

**2.** Verwendung von Verbindungen der allgemeinen Formel (II) gemäß Anspruch 1 zur Herstellung von (S)-Ketoprofen, dadurch gekennzeichnet, daß man die Verbindungen der allgemeinen Formel (II) in Gegenwart eines Katalysators der aus einem chiralen Übergangs-Metallkomplex besteht und gegebenenfalls in Gegenwart von inerten organischen Lösungsmitteln zu Verbindungen der allgemeinen Formel (I)

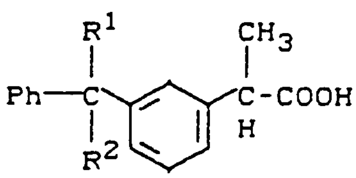

(I)

in welcher

$R^1$ und $R^2$ die oben angegebene Bedeutung haben, aber nicht gleichzeitig für Wasserstoff stehen,

hydriert und anschließend die Gruppe $CR^1R^2$ nach üblichen Methoden in eine Ketogruppe umwandelt.

**3.** Verfahren zur Herstellung von Verbindungen der allgemeinen Formel (IV), dadurch gekennzeichnet, daß man Verbindungen der allgemeinen Formel (V)

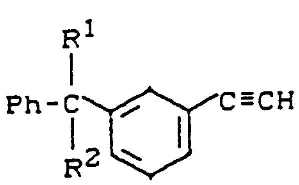

(V)

in welcher

$R^1$ und $R^2$ die im Anspruch 1 angegebene Bedeutung haben,

in Gegenwart von Palladium auf Aktivkohle als Katalysator und Kohlenmonoxid bei Drucken zwischen 1 und 200 bar mit einem Alkohol unter Zugabe von Mineralsäure, gegebenenfalls in Anwesenheit eines inerten aprotischen Lösungsmittels bei Temperaturen zwischen 25 und 160°C umsetzt.

**4.** Verfahren zur Herstellung von Verbindungen der allgemeinen Formel (III), dadurch gekennzeichnet, daß man Grignard-Verbindungen der Formel (VI)

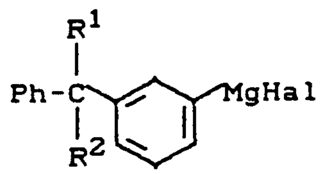

(VI)

in welcher

$R^1$ und $R^2$ die im Anspruch 1 angegebene Bedeutung haben und
Hal für Fluor, Jod, Chlor oder Brom steht,

mit einem Metall-pyruvat der allgemeinen Formel (VII)

$$Me^{\oplus}\ {}^{\ominus}OCC\text{-}CO\text{-}CH_3 \qquad (VII)$$

in welcher

Me für Alkali, Erdalkali (ein halbes Äquivalent) oder MgBr steht,

in Gegenwart von organischen Lösungsmitteln bei Temperaturen zwischen -10°C und +120°C umsetzt.

## Claims

**Claims for the following Contracting States : AT, BE, CH, DE, DK, FR, GB, IT, LI, LU, MC, NL, PT, SE**

**1.** (2)-Arylpropenoic acids of the general formula (II)

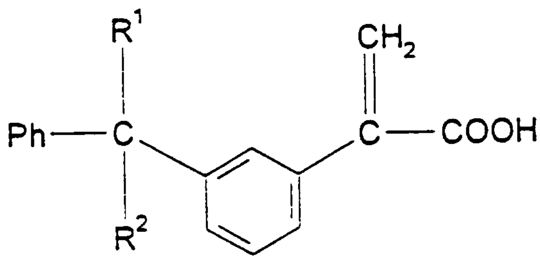

(II),

in which

$R^1$ and $R^2$ are identical or different and in each case represent hydrogen, hydroxyl, alkoxy having up to 10 C atoms, cycloalkoxy having 5 to 10 C atoms, aralkoxy having 7 to 12 C atoms or aryloxy having 6 to 10 C atoms, which is unsubstituted or substituted by alkyl or alkoxy having in each case 1 to 5 C atoms,

or
represent the group $O_2CR^3$, where $R^3$ is hydrogen, alkyl having up to 10 C atoms, cycloalkyl having 5 to 10 C atoms, aralkyl having 7 to 12 C atoms or aryl having 6 to 10 C atoms and unsubstituted or substituted by alkyl or alkoxy having in each case 1 to 5 C atoms,
or
represent the group $OSiR^4R^5R^6$, where $R^4$, $R^5$ and $R^6$, independently of each other, each have the meaning given for $R^3$
or
represent the group $NR^7R^8$, where $R^7$ and $R^8$, independently of each other, have the meaning given for $R^3$
or
represent a 2-tetrahydropyryl group
or

$R^1$ and $R^2$ together represent a group of the structure

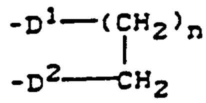

in which

$D^1$ and $D^2$, independently of each other, each represent oxygen, sulphur or the group $NR^3$, where $R^3$ has the meaning given above and

n represents 1, 2 or 3.

**2.** Compounds of the general formula (II) according to Claim 1, in which

$R^1$ and $R^2$ are identical or different and in each case represent hydrogen, hydroxyl, alkoxy having 1 to 4 C atoms, benzyloxy, acetyloxy, benzoyloxy, tri(alkyl)silyl having in each case 1 to 4 C atoms in the alkyl radicals, dialkylamino having in each case 1 to 4 C atoms in the alkyl radicals or 2-tetrahydropyryl

or

$R^1$ and $R^2$ together represent the group $-O(CH_2)_n-CH_2-D^2-$, where n represents 1, 2 or 3 and $D^2$ represents oxygen or alkylamine having 1 to 4 C atoms.

**3.** Process for the preparation of compounds of the general formula (II) according to Claim 1, characterised in that

A) compounds of the general formula (III)

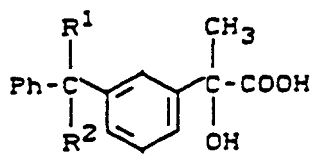

(III)

in which

$R^1$ and $R^2$ have the meaning given above,

are dehydrated by conventional methods
or

B) compounds of the formula (IV)

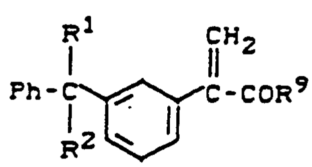

(IV)

in which

$R^1$ and $R^2$ have the meaning given above and

$R^9$ represents alkoxy having 1 to 10 C atoms, cycloalkoxy having 5 to 10 C atoms, aralkoxy having 7 to 12 C atoms or aryloxy having 6 to 10 C atoms, which is unsubstituted or substituted by alkyl

or alkoxy each having 1 to 5 C atoms,

are hydrolysed by conventional methods.

**4.** Use of compounds of the general formula (II) according to Claim 1 for the preparation of (S)-ketoprofen, characterised in that the compounds of the general formula (II) are hydrogenated in the presence of a catalyst which comprises a chiral transition metal complex and in the presence or absence of inert organic solvents to give compounds of the general formula (I)

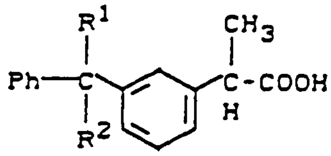

(I)

in which

$R^1$ and $R^2$ have the meaning given above, but do not simultaneously represent hydrogen,

and the $CR^1R^2$ group is subsequently converted into a keto group by conventional methods.

**5.** Compounds of the general formula (III)

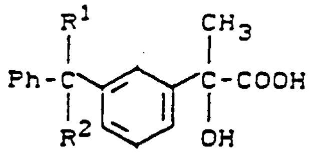

(III)

in which

$R^1$ and $R^2$ have the meaning given in Claim 1.

**6.** Compounds of the general formula (IV)

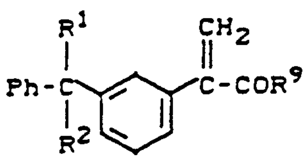

(IV)

in which

$R^1$ and $R^2$ have the meaning given in Claim 1 and

$R^9$ represents alkoxy having 1 to 10 C atoms, cycloalkoxy having 5 to 10 C atoms, aralkoxy having 7 to 12 C atoms or aryloxy having 6 to 10 C atoms, which is unsubstituted or substituted by alkyl or alkoxy each having 1 to 5 C atoms.

**7.** Process for the preparation of compounds of the general formula (IV), characterised in that compounds of the general formula (V)

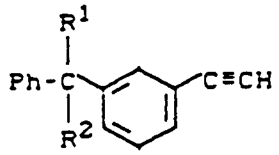

(V)

in which

$R^1$ and $R^2$ have the meaning given in Claim 1

are reacted in the presence of palladium on activated charcoal as catalyst and carbon monoxide at pressures between 1 and 200 bar with an alcohol and with addition of mineral acid, in the presence or absence of an inert aprotic solvent at temperatures between 25 and 160°C.

**8.** Process for the preparation of compounds of the general formula (III), characterised in that Grignard compounds of the formula (VI)

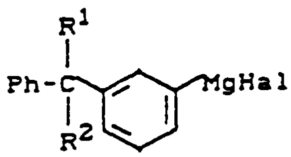

(VI)

in which

$R^1$ and $R^2$ have the meaning given in Claim 1 and

Hal represents fluorine, iodine, chlorine or bromine,

are reacted with a metal pyruvate of the general formula (VII)

$$Me^{\oplus}\ {}^{\ominus}OCC\text{-}CO\text{-}CH_3 \qquad (VII)$$

in which

Me represents an alkali metal, an alkaline earth metal (a half equivalent) or MgBr,

in the presence of organic solvents at temperatures between -10°C and +120°C.

**Claims for the following Contracting States : ES, GR**

**1.** Process for the preparation of compounds of the general formula (II)

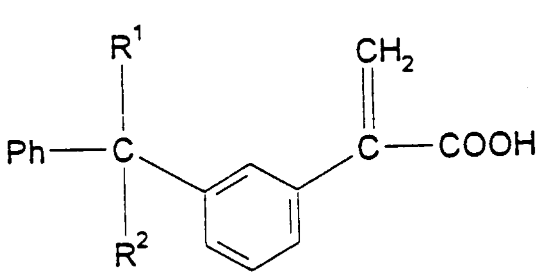

(II)

in which

$R^1$ and $R^2$ are identical or different and in each case represent hydrogen, hydroxyl, alkoxy having up to 10 C

atoms, cycloalkoxy having 5 to 10 C atoms, aralkoxy having 7 to 12 C atoms or aryloxy having 6 to 10 C atoms, which is unsubstituted or substituted by alkyl or alkoxy having in each case 1 to 5 C atoms,

or
represent the group $O_2CR^3$, where $R^3$ is hydrogen, alkyl having up to 10 C atoms, cycloalkyl having 5 to 10 C atoms, aralkyl having 7 to 12 C atoms or aryl having 6 to 10 C atoms and unsubstituted or substituted by alkyl or alkoxy having in each case 1 to 5 C atoms,
or
represent the group $OSiR^4R^5R^6$, where $R^4$, $R^5$ and $R^6$, independently of each other, each have the meaning given for $R^3$
or
represent the group $NR^7R^8$, where $R^7$ and $R^8$, independently of each other, have the meaning given for $R^3$
or
represent a 2-tetrahydropyryl group
or

$R^1$ and $R^2$ together represent a group of the structure

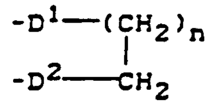

in which

$D^1$ and $D^2$, independently of each other, each represent oxygen, sulphur or the group $NR^3$, where $R^3$ has the meaning given above and

n represents 1, 2 or 3, characterised in that

A) compounds of the general formula (III)

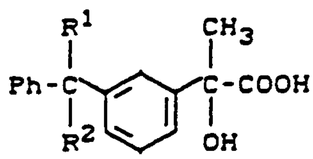

(III)

in which

$R^1$ and $R^2$ have the meaning given above,

are dehydrated by conventional methods
or

B) compounds of the formula (IV)

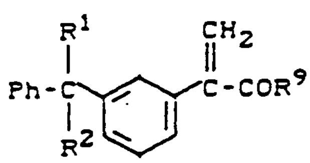

(IV)

in which

$R^1$ and $R^2$ have the meaning given above and

$R^9$ represents alkoxy having 1 to 10 C atoms, cycloalkoxy having 5 to 10 C atoms, aralkoxy having 7 to 12 C atoms or aryloxy having 6 to 10 C atoms, which is unsubstituted or substituted by alkyl or alkoxy each having 1 to 5 C atoms,

are hydrolysed by conventional methods.

**2.** Use of compounds of the general formula (II) according to Claim 1 for the preparation of (S)-ketoprofen, characterised in that the compounds of the general formula (II) are hydrogenated in the presence of a catalyst which comprises a chiral transition metal complex and in the presence or absence of inert organic solvents to give compounds of the general formula (I)

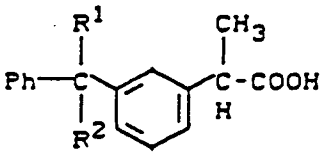

(I)

in which

$R^1$ and $R^2$ have the meaning given above, but do not simultaneously represent hydrogen,

and the $CR^1R^2$ group is subsequently converted into a keto group by conventional methods.

**3.** Process for the preparation of compounds of the general formula (IV), characterised in that compounds of the general formula (V)

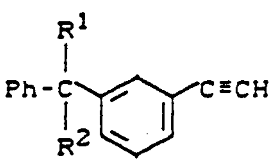

(V)

in which

$R^1$ and $R^2$ have the meaning given in Claim 1

are reacted in the presence of palladium on activated charcoal as catalyst and carbon monoxide at pressures between 1 and 200 bar with an alcohol and with addition of mineral acid, in the presence or absence of an inert aprotic solvent at temperatures between 25 and 160°C.

**4.** Process for the preparation of compounds of the general formula (III), characterised in that Grignard compounds of the formula (VI)

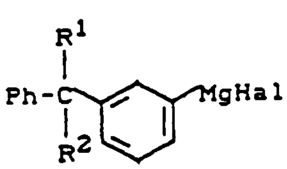

(VI)

in which

$R^1$ and $R^2$ have the meaning given in Claim 1 and

Hal represents fluorine, iodine, chlorine or bromine,

are reacted with a metal pyruvate of the general formula (VII)

$$Me^{\oplus}\ {}^{\ominus}OCC\text{-}CO\text{-}CH_3 \qquad (VII)$$

in which

Me represents an alkali metal, an alkaline earth metal (a half equivalent) or MgBr,

in the presence of organic solvents at temperatures between -10°C and +120°C.

## Revendications

**Revendications pour les Etats contractants suivants : AT, BE, CH, DE, DK, FR, GB, IT, LI, LU, MC, NL, PT, SE**

**1.** Acides (2)-arylpropénoïques de formule générale (II)

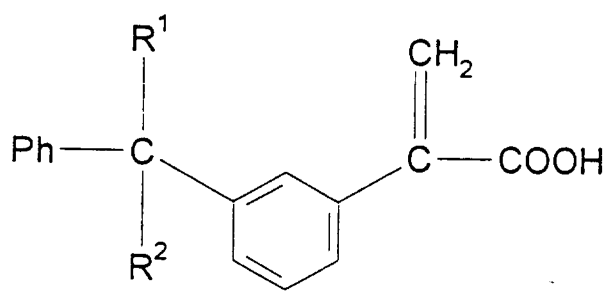

(II),

dans laquelle

$R^1$ et $R^2$ sont identiques ou différents et représentent chacun de l'hydrogène, un groupe hydroxy, alkoxy ayant jusqu'à 10 atomes de carbone, cycloalkoxy ayant 5 à 10 atomes de carbone, aralkoxy ayant 7 à 12 atomes de carbone ou aryloxy ayant 6 à 10 atomes de carbone, qui est substitué le cas échéant par un radical alkyle ou un radical alkoxy ayant chacun 1 à 5 atomes de carbone,

ou bien
le groupe $O_2CR^3$, dans lequel $R^3$ est de l'hydrogène, un groupe alkyle ayant jusqu'à 10 atomes de carbone, cycloalkyle ayant 5 à 10 atomes de carbone, aralkyle ayant 7 à 12 atomes de carbone ou aryle ayant 6 à 10 atomes de carbone, le cas échéant substitué avec un radical alkyle ou un radical alkoxy ayant chacun 1 à 5 atomes de carbone,
ou bien
le groupe $OSiR^4R^5R^6$, dans lequel $R^4$, $R^5$ et $R^6$ ont chacun, indépendamment des autres, la définition indiquée pour $R^3$
ou bien
le groupe $NR^7R^8$, dans lequel $R^7$ et $R^8$ ont, indépendamment l'un de l'autre, la définition indiquée pour $R^3$
ou bien
un groupe 2-tétrahydropyrryle
ou bien

$R^1$ et $R^2$ forment en commun un groupement de structure

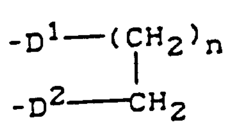

dans laquelle

$D^1$ et $D^2$ désignent chacun, indépendamment l'un de l'autre, l'oxygène, le soufre ou le groupe $NR^3$ dans lequel $R^3$ a la définition indiquée ci-dessus et

n a la valeur 1, 2 ou 3.

**2.** Composés de formule générale (II) suivant la revendication 1, dans laquelle

$R^1$ et $R^2$ sont identiques ou différents et représentent chacun de l'hydrogène, un groupe hydroxy, un groupe alkoxy ayant 1 à 4 atomes de carbone, benzyloxy, acétyloxy, benzoyloxy, tri(alkyl)silyle ayant dans chaque cas 1 à 4 atomes de carbone dans les restes alkyle, un groupe dialkylamino ayant 1 à 4 atomes de carbone dans chacun des restes alkyle ou le groupe 2-tétrahydropyrryle,

ou bien

$R^1$ et $R^2$ forment ensemble le groupe $-O(CH_2)_n-CH_2-D^2-$, dans lequel n a la valeur 1, 2 ou 3 et $D^2$ représente de l'oxygène ou un groupe alkylamino ayant 1 à 4 atomes de carbone.

**3.** Procédé de production de composés de formule générale (II) suivant la revendication 1, caractérisé en ce que :

A) on déshydrate par des procédés classiques des composés de formule générale (III)

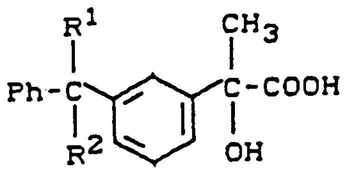

(III)

dans laquelle

$R^1$ et $R^2$ ont la définition indiquée ci-dessus,

ou bien

B) on hydrolyse par des procédés classiques des composés de formule (IV)

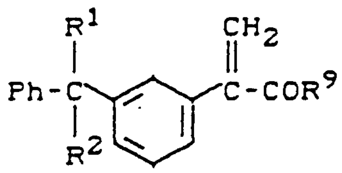

(IV)

dans laquelle

$R^1$ et $R^2$ ont la définition indiquée ci-dessus et

$R^9$ est un groupe alkoxy ayant 1 à 10 atomes de carbone, cycloalkoxy ayant 5 à 10 atomes de carbone, aralkoxy ayant 7 à 12 atomes de carbone ou aryloxy ayant 6 à 10 atomes de carbone, qui est substitué le cas échéant par un groupe alkyle ou un groupe alkoxy ayant chacun 1 à 5 atomes de carbone.

**4.** Utilisation de composés de formule générale (II) suivant la revendication 1 pour la production de (S)-cétoprofène, caractérisée en ce qu'on hydrogène les composés de formule générale (II) en présence d'un catalyseur qui est constitué d'un complexe chiral d'un métal de transition et, le cas échéant, en présence de solvants organiques inertes, pour obtenir des composés de formule générale (I)

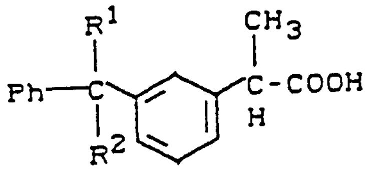

(I)

dans laquelle

$R^1$ et $R^2$ ont la définition indiquée ci-dessus, mais ne représente pas simultanément de l'hydrogène,

puis on transforme le groupe $CR^1R^2$ par des procédés classiques en un groupe céto.

**5.** Composés de formule générale (III)

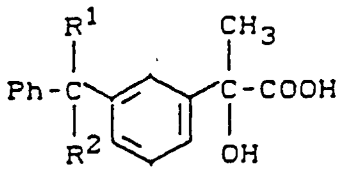

(III)

dans laquelle

$R^1$ et $R^2$ ont la définition indiquée dans la revendication 1.

**6.** Composés de formule générale (IV)

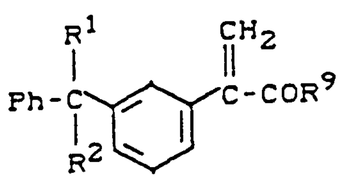

(IV)

dans laquelle

$R^1$ et $R^2$ ont la définition indiquée dans la revendication 1 et
$R^9$ est un groupe alkoxy ayant 1 à 10 atomes de carbone, cycloalkoxy ayant 5 à 10 atomes de carbone, aralkoxy ayant 7 à 12 atomes de carbone ou aryloxy ayant 6 à 10 atomes de carbone, qui est substitué le cas échéant par un radical alkyle ou un radical alkoxy ayant chacun 1 à 5 atomes de carbone.

**7.** Procédé de production de composés de formule générale (IV), caractérisé en ce qu'on fait réagir des composés de formule générale (V)

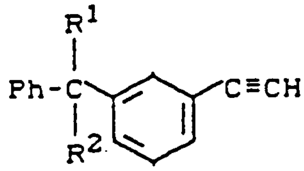

(V)

dans laquelle

$R^1$ et $R^2$ ont la définition indiquée dans la revendication 1,

en présence de palladium sur du charbon actif utilisé comme catalyseur et d'oxyde de carbone à des pressions comprises entre 1 et 200 bars, avec un alcool additionné d'un acide minéral, le cas échéant en présence d'un solvant aprotique inerte, à des températures comprises entre 25 et 160°C.

**8.** Procédé de production de composés de formule générale (III), caractérisé en ce qu'on fait réagir des composés de Grignard de formule (VI)

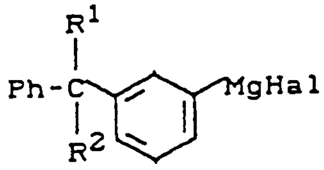

(VI)

dans laquelle

$R^1$ et $R^2$ ont la définition indiquée dans la revendication 1 et
Hal représente du fluor, de l'iode, du chlore ou du brome,

avec un pyruvate métallique de formule générale (VII)

$$Me^{\oplus}\ {}^{\ominus}OCC\text{-}CO\text{-}CH_3 \qquad (VII)$$

dans laquelle

Me représente un métal alcalin, un métal alcalino-terreux (un demi-équivalent) ou MgBr,

en présence de solvants organiques, à des températures comprises entre -10°C et +120°C.

**Revendications pour les Etats contractants suivants : ES, GR**

**1.** Procédé de production de composés de formule générale (II)

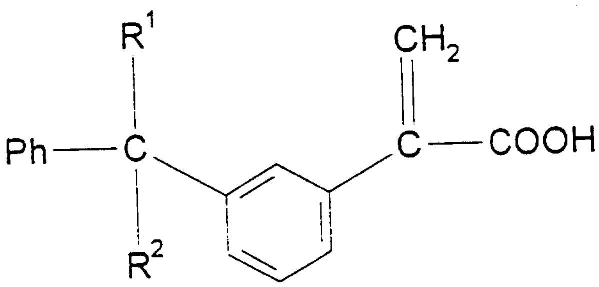

(II)

dans laquelle

$R^1$ et $R^2$ sont identiques ou différents et représentent chacun de l'hydrogène, un groupe hydroxy, alkoxy ayant jusqu'à 10 atomes de carbone, cycloalkoxy ayant 5 à 10 atomes de carbone, aralkoxy ayant 7 à 12 atomes de carbone ou aryloxy ayant 6 à 10 atomes de carbone, qui est substitué le cas échéant par un radical alkyle ou un radical alkoxy ayant chacun 1 à 5 atomes de carbone,

ou bien
le groupe $O_2CR^3$, dans lequel $R^3$ est de l'hydrogène, un groupe alkyle ayant jusqu'à 10 atomes de carbone, cycloalkyle ayant 5 à 10 atomes de carbone, aralkyle ayant 7 à 12 atomes de carbone ou aryle ayant 6 à 10 atomes de carbone, le cas échéant substitué avec un radical alkyle ou un radical alkoxy ayant chacun 1 à 5 atomes de carbone,

ou bien
le groupe $OSiR^4R^5R^6$, dans lequel $R^4$, $R^5$ et $R^6$ ont chacun, indépendamment des autres, la définition indiquée pour $R^3$
ou bien
le groupe $NR^7R^8$, dans lequel $R^7$ et $R^8$ ont, indépendamment l'un de l'autre, la définition indiquée pour $R^3$
ou bien
un groupe 2-tétrahydropyrryle
ou bien

$R^1$ et $R^2$ forment en commun un groupement de structure

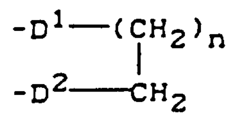

dans laquelle

$D^1$ et $D^2$ désignent chacun, indépendamment l'un de l'autre, l'oxygène, le soufre ou le groupe $NR^3$ dans lequel $R^3$ a la définition indiquée ci-dessus et
<u>n</u> a la valeur 1, 2 ou 3,

caractérisé en ce que :

A) on déshydrate par des procédés classiques des composés de formule générale (III)

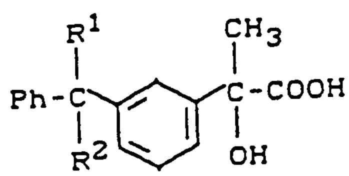

(III)

dans laquelle

$R^1$ et $R^2$ ont la définition indiquée ci-dessus,

ou bien
B) on hydrolyse par des procédés classiques des composés de formule (IV)

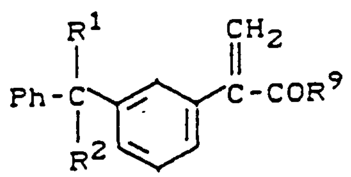

(IV)

dans laquelle

$R^1$ et $R^2$ ont la définition indiquée ci-dessus et
$R^9$ est un groupe alkoxy ayant 1 à 10 atomes de carbone, cycloalkoxy ayant 5 à 10 atomes de carbone, aralkoxy ayant 7 à 12 atomes de carbone ou aryloxy ayant 6 à 10 atomes de carbone, qui est substitué le cas échéant par un groupe alkyle ou un groupe alkoxy ayant chacun 1 à 5 atomes de carbone.

**2.** Utilisation de composés de formule générale (II) suivant la revendication 1 pour la production de (S)-cétoprofène, caractérisée en ce qu'on hydrogène les composés de formule générale (II) en présence d'un catalyseur qui est constitué d'un complexe chiral d'un métal de transition et, le cas échéant, en présence de solvants organiques inertes, pour obtenir des composés de formule générale (I)

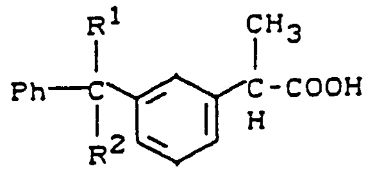

(I)

dans laquelle

$R^1$ et $R^2$ ont la définition indiquée ci-dessus, mais ne représente pas simultanément de l'hydrogène,

puis on transforme le groupe $CR^1R^2$ par des procédés classiques en un groupe céto.

**3.** Procédé de production de composés de formule générale (IV), caractérisé en ce qu'on fait réagir des composés de formule générale (V)

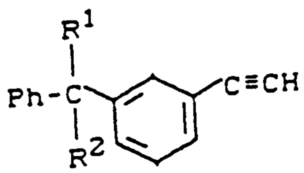

(V)

dans laquelle

$R^1$ et $R^2$ ont la définition indiquée dans la revendication 1,

en présence de palladium sur du charbon actif utilisé comme catalyseur et d'oxyde de carbone à des pressions comprises entre 1 et 200 bars, avec un alcool additionné d'un acide minéral, le cas échéant en présence d'un solvant aprotique inerte, à des températures comprises entre 25 et 160°C.

**4.** Procédé de production de composés de formule générale (III), caractérisé en ce qu'on fait réagir des composés de Grignard de formule (VI)

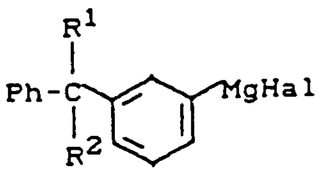

(VI)

dans laquelle

$R^1$ et $R^2$ ont la définition indiquée dans la revendication 1 et
Hal représente du fluor, de l'iode, du chlore ou du brome,

avec un pyruvate métallique de formule générale (VII)

$$Me^{\oplus}\ {}^{\ominus}OCC\text{-}CO\text{-}CH_3 \qquad (VII)$$

dans laquelle

Me représente un métal alcalin, un métal alcalino-terreux (un demi-équivalent) ou MgBr,

en présence de solvants organiques, à des températures comprises entre -10°C et +120°C.